(19) Europäisches Patentamt
European Patent Office
Office européen des brevets

(11) EP 4 763 221 A1

(12) EUROPEAN PATENT APPLICATION
published in accordance with Art. 153(4) EPC

(43) Date of publication:
**24.06.2026 Bulletin 2026/26**

(21) Application number: **24853885.2**

(22) Date of filing: **16.08.2024**

(51) International Patent Classification (IPC):
***A61K 39/395*** *(2006.01)* ***C07K 5/062*** *(2006.01)*
***A61K 31/4745*** *(2006.01)* ***A61K 31/48*** *(2006.01)*
***A61P 35/00*** *(2006.01)*

(52) Cooperative Patent Classification (CPC):
**A61K 31/4745; A61K 31/48; A61K 38/07; A61K 39/395; A61K 47/68; A61P 35/00; C07K 5/021; C07K 5/06017; C07K 16/32**

(86) International application number:
**PCT/CN2024/112677**

(87) International publication number:
**WO 2025/036480 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.08.2023 CN 202311037231**

(71) Applicant: **Akeso Biopharma Co., Ltd.**
**Zhongshan, Guangdong 528437 (CN)**

(72) Inventors:
- **LI, Baiyong**
  **Zhongshan, Guangdong 528437 (CN)**
- **XIA, Yu**
  **Zhongshan, Guangdong 528437 (CN)**
- **WANG, Zhongmin**
  **Zhongshan, Guangdong 528437 (CN)**
- **ZHANG, Peng**
  **Zhongshan, Guangdong 528437 (CN)**

(74) Representative: **Berggren Oy**
**P.O. Box 16**
**Fabianinkatu 21**
**00101 Helsinki (FI)**

Remarks:
The complete document including Reference Table(s) and the Sequence Listing(s) can be downloaded from the EPO website

# (54) COMPOUND AND ANTIBODY-DRUG CONJUGATE COMPRISING SAME

(57) Provide are a compound and an antibody-drug conjugate comprising same, which belong to the field of biomedicine. Further provided are a pharmaceutical composition comprising the antibody-drug conjugate and the use thereof. Specifically, the antibody-drug conjugate is an anti-HER3 antibody-drug conjugate. The antibody-drug conjugate has good stability and biological activity.

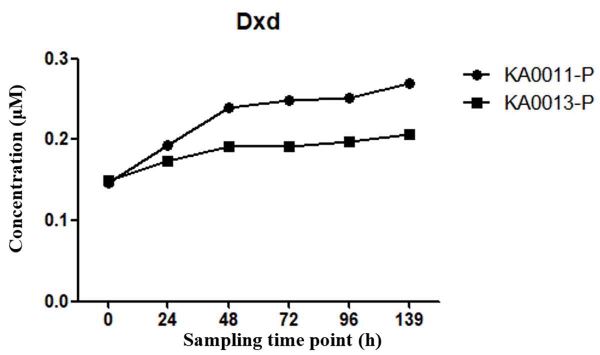


FIG. 4

EP 4 763 221 A1

## Description

CROSS-REFERENCE TO RELATED APPLICATIONS

The present application is based on Chinese Patent Application No. 202311037231.5 filed on August 16, 2023 and claims priority thereto, which is incorporated herein by reference in its entirety.

TECHNICAL FIELD

The present disclosure belongs to the field of biomedicines, and relates to a compound and an antibody-drug conjugate comprising same. The present disclosure also relates to a pharmaceutical composition comprising the antibody-drug conjugate and use thereof. Specifically, the antibody-drug conjugate is an anti-HER3 antibody-drug conjugate.

BACKGROUND

Cancer is the leading bane threatening human life and health. Traditional treatment methods include surgery, chemotherapy, radiotherapy, etc., but these methods usually exert toxic and side effects on normal cells while killing cancer cells, resulting in a narrow therapeutic window. Immunotherapy is a significant approach in cancer treatment, which typically leverages the body’s own immune mechanisms to combat cancer cells, and is a preferred treatment scheme for cancer patients with small early lesions due to its unique advantages of high targeting property and few side effects. However, for advanced solid tumors, immunotherapy has limited efficacy and requires combination with other methods to jointly combat cancer cells (Li Yunfeng, Xin Jie, Li Jing, et al., Research progress of linkage technology of antibody-drug conjugates[J]. Chinese Journal of New Drugs and Clinical Remedies, 2022, 41(12): 10).

Antibody-drug conjugates (ADCs) are an emerging class of macromolecular targeted drugs that combine the powerful killing effect of traditional small-molecule chemotherapy with the tumor-targeting property of antibody drugs. An ADC consists of three main moieties: an antibody responsible for selectively recognizing a cancer cell surface antigen, a payload responsible for killing cancer cells, and a linker linking the antibody and the payload. After the ADC drug enters the blood circulation, its antibody moiety specifically binds to an antigen on the surface of tumor cells, and the complex formed by the binding enters the cells through receptor-mediated endocytosis (which can be classified into four types: clathrin-mediated endocytosis, cave-like invagination, macropinocytosis, and clathrin- and caveolin-independent endocytosis). Cleavable linkers are sensitive to environmental factors in tumor cells and can be cleaved by specific pH environments, proteases, or certain chemical substances. ADC drugs carrying the non-cleavable linkers are digested by lysosomes, leading to the release of the drug. Small-molecule payloads of certain ADC drugs can penetrate cell membranes to further kill surrounding cancer cells, achieving a bystander killing effect (Khongorzul, Puregmaa, et al., "Antibody-Drug Conjugates: A Comprehensive Review". Mol Cancer Res 1(2020); Kostova Vesela, Désos Patrice, Starck Jérôme-Benoît et al., The Chemistry Behind ADCs.[J]. Pharmaceuticals (Basel), 2021, 14: undefined.).

The accumulation of payloads within tumors is limited, thus requiring tumor killing at sub-nanomolar concentrations. In addition, payloads need to have good stability to maintain structural integrity and activity in the circulatory system and lysosomes. Common payloads mainly include tubulin inhibitors and DNA damaging agents. Tubulin inhibitors achieve the purpose of killing tumor cells and inhibiting the rapid proliferation of tumor cells by inhibiting the generation and aggregation of tubulin. Tubulin inhibitors commonly used for ADC construction include maytansines, auristatins, tubulysins, and the like. DNA damaging agents are classified into three major categories based on different mechanisms of action: DNA double-strand breakers, DNA intercalators, and DNA alkylating agents. DNA is critical in the growth and proliferation process of cells. By disrupting DNA, tumor cells can be efficiently killed and the rapid proliferation of the tumor cells can be inhibited. DNA damaging agents commonly used for ADC construction include pyrrolobenzodiazepines, indolochlorobenzodiazepines (e.g., pyrrolo[2,1-c][1,4]benzodiazepine, PBD), duocarmycins, camptothecin (CPT) and derivatives thereof (e.g., topoisomerase I inhibitors Dxd and SN-38), calicheamicins, and the like (Dan Nimoy, Setua Saini, Kashyap Vivek K et al., Antibody-Drug Conjugates for Cancer Therapy: Chemistry to Clinical Implications.[J]. Pharmaceuticals(Basel), 2018, 11: undefined.).

HER3 is a member of the human epidermal growth factor receptor (HER) family and consists of three moieties: an extracellular ligand-binding region, an α-helical transmembrane region, and an intracellular tyrosine kinase region. HER3 is abnormally overexpressed in a variety of invasive tumors, including breast tumor, non-small cell lung cancer (NSCLC), metastatic colon cancer, head and neck cancer, pancreatic cancer, ovarian cancer, clear cell sarcoma, gastric cancer, skin cancer, and the like. HER3 can bind to the ligand neuregulin (NRG). Although HER3 lacks intrinsic tyrosine kinase activity, it can form a heterodimer with HER2 to induce the crosslinking and phosphorylation of highly conserved intracellular kinase residues, that is, a process where one receptor phosphorylates specific tyrosine residues on the other, thereby recruiting and activating downstream proteins. This process directly activates the PI3K/AKT pathway, triggering a signal

cascade that promotes the occurrence and progression of tumor cells.

In summary, the development of HER3-targeted ADC drugs with significant efficacy and low toxic and side effects has broad prospects.

SUMMARY

Through intensive studies and creative efforts, the inventors have prepared a compound capable of serving as a linker and a payload, and have further prepared an antibody-drug conjugate. The prepared antibody-drug conjugate has good stability and/or biological activity. The present disclosure is detailed below:

One aspect of the present disclosure relates to a compound or a pharmaceutically acceptable salt thereof, wherein the compound comprises a compound of Formula III, n alanine residues, and a compound of Formula IV that are sequentially linked,

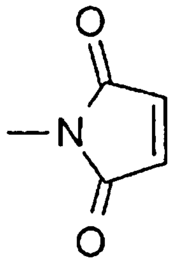

Formula III,

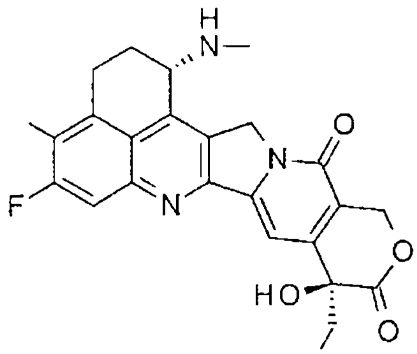

Formula IV,

wherein

n is 2, 3, 4, 5, or 6;

any two of the compound of Formula III, the n alanine residues, and the compound of Formula IV are independently either linked directly (e.g., via a chemical bond) or via a chemical group.

The pharmaceutically acceptable salt of the compound of the present disclosure includes conventional salts formed with pharmaceutically acceptable inorganic or organic acids, or inorganic or organic bases. Examples of suitable acid addition salts include salts formed with hydrochloric acid, hydrobromic acid, sulfuric acid, phosphoric acid, nitric acid, perchloric acid, fumaric acid, acetic acid, propionic acid, succinic acid, glycolic acid, formic acid, lactic acid, maleic acid, tartaric acid, citric acid, pamoic acid, malonic acid, hydroxymaleic acid, phenylacetic acid, glutamic acid, benzoic acid, salicylic acid, fumaric acid, toluenesulfonic acid, methanesulfonic acid, naphthalene-2-sulfonic acid, benzenesulfonic acid, hydroxynaphthoic acid, hydroiodic acid, malic acid, tannic acid, and the like. Examples of suitable base addition salts include salts formed with sodium, lithium, potassium, magnesium, aluminum, calcium, zinc, N,N'-dibenzylethylenediamine, chloroprocaine, choline, diethanolamine, ethylenediamine, N-methylglucamine, procaine, and the like.

In some embodiments of the present disclosure, provided is the compound or the pharmaceutically acceptable salt thereof, having a structural formula represented by Formula I below,

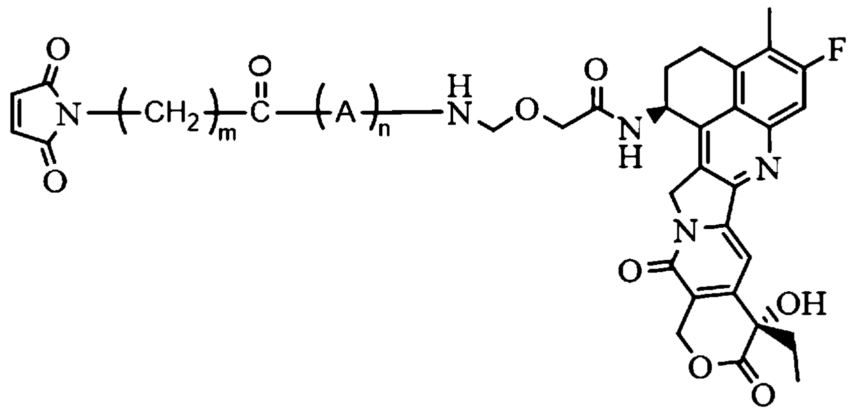

Formula I

wherein

A represents alanine (alanine residue);
m is 2, 3, 4, 5, 6, 7, 8, 9, 10, 11, or 12;
n is 2, 3, 4, 5, or 6.

In some embodiments of the present disclosure, provided is the compound or the pharmaceutically acceptable salt, wherein the compound is represented by Formula II below,

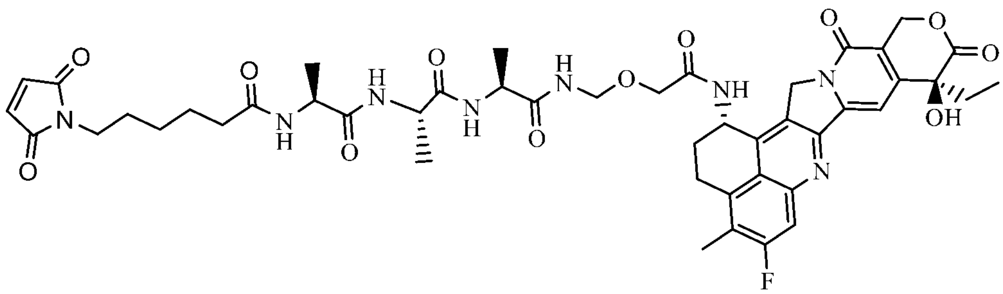

Formula II

Another aspect of the present disclosure relates to an antibody-drug conjugate (ADC) comprising an antibody or an antigen-binding fragment thereof, a linker, and a payload,

wherein the linker and the payload are the compound or the pharmaceutically acceptable salt thereof according to any one of the aspects of the present disclosure;
preferably, the linker is linked to the antibody or the antigen-binding fragment thereof via one or more thioether bonds;
preferably, the linker forms a thioether bond with a sulfur atom at a disulfide bond position of the hinge portion of the antibody.

Without being bound by theory, the maleimide-N-yl undergoes an alkylation reaction with the sulfhydryl group in the antibody to form a stable thioether bond.
In some embodiments of the present disclosure, provided is the antibody-drug conjugate, wherein the antibody is an IgG antibody, preferably an IgG1 antibody.
In some embodiments of the present disclosure, provided is the antibody-drug conjugate, wherein the antibody is an anti-HER3 antibody.
In some embodiments of the present disclosure, provided is the antibody-drug conjugate, wherein the antibody comprises a heavy chain variable region and a light chain variable region,
wherein

the heavy chain variable region comprises an HCDR1 set forth in SEQ ID NO: 1, an HCDR2 set forth in SEQ ID NO: 2,

and an HCDR3 set forth in SEQ ID NO: 3;
the light chain variable region comprises an LCDR1 set forth in SEQ ID NO: 4, an LCDR2 set forth in SEQ ID NO: 5, and an LCDR3 set forth in SEQ ID NO: 6.

In some embodiments of the present disclosure, provided is the antibody-drug conjugate, wherein the heavy chain variable region comprises an amino acid sequence set forth in SEQ ID NO: 7, and the light chain variable region comprises an amino acid sequence set forth in SEQ ID NO: 9.
In some embodiments of the present disclosure, provided is the antibody-drug conjugate, wherein a heavy chain constant region of the antibody is an Ig gamma-1 chain C region or an Ig gamma-4 chain C region, and a light chain constant region is an Ig kappa chain C region.
In some embodiments of the present disclosure, provided is the antibody-drug conjugate, wherein the heavy chain constant region of the antibody comprises an amino acid sequence set forth in SEQ ID NO: 11, and the light chain constant region comprises an amino acid sequence set forth in SEQ ID NO: 13.
In some embodiments of the present disclosure, provided is the antibody-drug conjugate, wherein an average number of linker-payloads conjugated to each antibody molecule is 1-8, 2-8, 3-8, 4-8, 5-8, 6-8, or 7-8.
In some embodiments of the present disclosure, provided is the antibody-drug conjugate, wherein the antigen-binding fragment is selected from Fab, Fab', $F(ab')_2$, Fd, Fv, dAb, a complementarity determining region fragment, a single chain fragment variable, a humanized antibody, a chimeric antibody, and a diabody.
In some embodiments of the present disclosure, provided is the antibody-drug conjugate, wherein, the heavy chain constant region of the antibody is a heavy chain constant region of human IgG1, IgG2, IgG3 or IgG4 or a variant thereof, preferably a heavy chain constant region of human IgG1; the light chain constant region is an Ig kappa chain C region.
In some embodiments of the present disclosure, provided is the antibody-drug conjugate, which has ADCC activity.
In some embodiments of the present disclosure, provided is the antibody-drug conjugate, wherein
"An $EC_{50}$ for binding of the antibody-drug conjugate to an antigen targeted by the antibody-drug conjugate" is identical to or substantially identical to "an $EC_{50}$ for binding of an antibody or an antigen-binding fragment thereof comprised in the antibody-drug conjugate to the same antigen". "Substantially identical" herein may mean, for example: the difference between "an $EC_{50}$ for binding of the antibody-drug conjugate to an antigen targeted by the antibody-drug conjugate" and "an $EC_{50}$ for binding of an antibody or an antigen-binding fragment thereof comprised in the antibody-drug conjugate to the same antigen" is within $\pm$10%, within $\pm$15%, within $\pm$20%, within $\pm$25%, within $\pm$30%, within $\pm$35%, within $\pm$40%, or within $\pm$45%.
The antibody-drug conjugate of the present disclosure can be used for treating or preventing a tumor;

preferably, the tumor is an HER3-positive tumor;
preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon cancer, rectal cancer, uterine cancer, salivary gland cancer, vulvar cancer, thyroid cancer, anal cancer, and penile cancer;
preferably, the lung cancer is non-small cell lung cancer (NSCLC);
preferably, the colon cancer is metastatic colon cancer.

An antibody-drug conjugate can be prepared by reacting a compound with an antibody or a reactive derivative thereof and conjugating the compound or the pharmaceutically acceptable salt thereof of the present disclosure to the antibody by forming a thioether bond in a disulfide bond moiety present in the hinge portion of the antibody.
Yet another aspect of the present disclosure relates to a pharmaceutical composition comprising the antibody-drug conjugate according to any one of the aspects of the present disclosure, and one or more pharmaceutically acceptable auxiliary materials.
Yet another aspect of the present disclosure relates to use of the antibody-drug conjugate according to any one of the aspects of the present disclosure in the preparation of a medicament for treating or preventing a tumor;

preferably, the tumor is an HER3-positive tumor;
preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon

cancer, rectal cancer, uterine cancer, salivary gland cancer, vulvar cancer, thyroid cancer, anal cancer, and penile cancer;
preferably, the lung cancer is non-small cell lung cancer (NSCLC);
preferably, the colon cancer is metastatic colon cancer.

Yet another aspect of the present disclosure relates to a method for treating or preventing a tumor, comprising a step of administering to a subject in need an effective amount of the antibody-drug conjugate according to any one of the aspects of the present disclosure;

preferably, the tumor is an HER3-positive tumor;
preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon cancer, rectal cancer, uterine cancer, salivary gland cancer, vulvar cancer, thyroid cancer, anal cancer, and penile cancer;
preferably, the lung cancer is non-small cell lung cancer (NSCLC);
preferably, the colon cancer is metastatic colon cancer.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, the term $EC_{50}$ refers to the concentration for 50% of maximal effect, i.e., the concentration that can cause 50% of the maximal effect.

As used herein, the term "no ADCC activity" refers to the situation where, as detected by existing instruments and equipment or detection methods (such as the method described in Example 9), no ADCC activity is observed, ADCC activity is undetectable, or the activity falls below the detection limit of the instruments, equipment and detection methods.

As used herein, the term "antibody" refers to an immunoglobulin molecule that generally consists of two pairs of polypeptide chains (each pair with one "light" (L) chain and one "heavy" (H) chain). Antibody light chains can be classified into $\kappa$ and $\lambda$ light chains. Heavy chains can be classified into $\mu$, $\delta$, $\gamma$, $\alpha$, or $\varepsilon$. Isotypes of antibodies are defined as IgM, IgD, IgG, IgA, and IgE. In light chains and heavy chains, the variable region and constant region are linked via a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), with conservative regions called framework regions (FRs) interspersed therebetween. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Chothia & Lesk J. Mol. Biol., 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The variable regions of the light and heavy chains determine antigen binding; the variable region of each chain contains three hypervariable regions called complementarity determining regions (CDRs), wherein the CDRs of the heavy chain (H) include HCDR1, HCDR2, and HCDR3, and the CDRs of the light chain (L) include LCDR1, LCDR2, and LCDR3. In the present disclosure, the CDRs are defined by the IMGT numbering system. See Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1):D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for

natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained using hybridoma technology first reported by Kohler et al. (Köhler G, Milstein C., Continuous cultures of fused cells secreting antibody of predefined specificity [J]. Nature, 1975; 256(5517):495), but can also be obtained using recombinant DNA technology (see, e.g., U.S. Patent 4,816,567).

As used herein, the term "humanized antibody" refers to an antibody or an antibody fragment obtained when all or a part of the CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402. In some cases, the antigen-binding fragments of the antibodies are diabodies, in which the VH and VL domains are expressed on a single polypeptide chain. However, the linker used is too short to allow the pairing of the two domains on the same chain. Thus, the domains are forced to pair with the complementary domains on the other chain, and two antigen-binding sites are generated (see, e.g., Holliger P. et al., Proc. Natl. Acad. Sci. USA, 1993; 90:6444-6448 and Poljak R.J. et al., Structure, 1994; 2:1121-1123). As used herein, the term "single chain fragment variable (ScFv)" refers to a molecule comprising an antibody heavy chain variable region (VH) and an antibody light chain variable region (VL) linked via a linker. The VL and VH domains are paired to form a monovalent molecule via a linker enabling production as a single polypeptide chain (see, e.g., Bird et al., Science, 1988; 242:423-426 and Huston et al., Proc. Natl. Acad. Sci. USA, 1988; 85:5879-5883). Such scFv molecules may have the following general structures: $NH_2$-VL-linker fragment-VH-COOH or $NH_2$-VH-linker fragment-VL-COOH. A suitable linker in the prior art consists of a repeated GGGGS (SEQ ID NO: 15) amino acid sequence or a variant thereof. For example, a linker having the amino acid sequence (GGGGS)4 (SEQ ID NO: 16) may be used, but variants thereof may also be used (Holliger et al., Proc. Natl. Acad. Sci. USA, 1993; 90: 6444-6448). Other linkers that can be used in the present disclosure are described in Alfthan et al., Protein Eng., 1995; 8: 725-731; Choi et al., Eur. J. Immunol., 2001; 31: 94-106; Hu et al., Cancer Res., 1996; 56: 3055-3061; Kipriyanov et al., J. Mol. Biol., 1999; 293: 41-56; and Roovers et al., Cancer Immunology, Immunotherapy, 2001, 50(1): 51-59.

As used herein, the term "antibody moiety" refers to an antibody moiety in an antibody-drug conjugate, which, in certain specific embodiments, is linked to an intermediate linker moiety via a specific functional group, and can specifically bind to an antigen.

As used herein, the term "linker" refers to a moiety that links an antibody to a cytotoxin, and is classified into cleavable and non-cleavable forms. Cleavable linkers mainly include sensitive chemical bonds, and can facilitate the cleavage between the linker and the drug according to specific chemical species (such as glutathione and acidity or basicity) or different enzyme concentrations in the internal environment of the body, and are mainly linked in the form of hydrazone bonds, disulfide bonds, or polypeptide linkages. Non-cleavable linkers have no built-in chemical bond that can trigger cleavage, and thus antibodies need to be converted into amino acids by proteolysis in cancer cells to release cytotoxic drugs carrying the linkers and amino acid fragments, which are primarily linked in the form of thioethers.

As used herein, the term "payload" refers to a cytotoxin molecule mainly responsible for performing a cell killing function in an antibody-drug conjugate. The payload is linked to an intermediate linker moiety by chemical synthesis, enters tumor cells through internalization, and releases the cytotoxin molecule under the action of lysosomes, thereby exerting an anti-tumor effect. Common targets are DNA in the nucleus and tubulin in the cytoplasm.

As used herein, the term "DAR (drug-to-antibody ratio)" refers to an average number of small-molecule drugs conjugated to each antibody molecule, which is one of the important quality attributes of ADCs. According to the chemical properties and conjugation mode (lysine conjugation, cysteine conjugation, glycosyl conjugation, etc.) of the linker and the small-molecule cytotoxin, the common characterization and analysis methods include ultraviolet-visible spectrophotometry, hydrophobic chromatography, reversed-phase chromatography, and mass spectrometry (MS).

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those

skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as $\lambda$ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, GS cells, COS cells, NS0 cells, HeLa cells, BHK cells, HEK293 cells, or human cells.

As used herein, the term "specifically bind" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (KD) of less than about $10^{-5}$ M, such as less than about $10^{-6}$ M, $10^{-7}$ M, $10^{-8}$ M, $10^{-9}$ M, or $10^{-10}$ M, or less.

As used herein, the term "KD" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, antibodies bind to antigens with a dissociation equilibrium constant (KD) of less than about $10^{-5}$ M, such as less than about $10^{-6}$ M, $10^{-7}$ M, $10^{-8}$ M, $10^{-9}$ M or $10^{-10}$ M or less. KD can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably. Besides, in the present disclosure, amino acids are generally represented by single-letter and three-letter abbreviations known in the art. For example, alanine can be represented by A or Ala.

As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the development of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

Beneficial Effects of the Present Disclosure

The present disclosure achieves one or more of the following technical effects:

(1) The antibody-drug conjugates, such as anti-HER3 antibody-drug conjugate KA0013, prepared using the compound of the present disclosure have good biological activity.
(2) The antibody-drug conjugates, such as anti-HER3 antibody-drug conjugate KA0013, prepared using the compound of the present disclosure can effectively release the payload moiety.
(3) The anti-HER3 antibody-drug conjugate KA0013 of the present disclosure can effectively bind to HER3.
(4) The antibody-drug conjugates, such as anti-HER3 antibody-drug conjugate KA0013, prepared using the compound of the present disclosure have internalization activity.
(5) The antibody-drug conjugates, such as anti-HER3 antibody-drug conjugate KA0013, prepared using the compound of the present disclosure have good stability in plasma.
(6) The antibody-drug conjugates, such as anti-HER3 antibody-drug conjugate KA0013, prepared using the compound of the present disclosure can specifically kill tumor cells.
(7) The antibody-drug conjugates, such as HER3 antibody-drug conjugate KA0013, prepared using the compound of

the present disclosure have ADCC activity.

(8) The antibody-drug conjugates, such as anti-HER3 antibody-drug conjugate KA0013, prepared using the compound of the present disclosure can effectively inhibit tumor growth *in vivo.*

(9) The antibody-drug conjugates, such as HER3 antibody-drug conjugate KA0013, prepared using the compound of the present disclosure have good safety.

(10) The antibody-drug conjugates, such as HER3 antibody-drug conjugate KA0013, prepared using the compound of the present disclosure have a bystander killing effect.

BRIEF DESCRIPTION OF THE DRAWINGS

FIG. 1: Assay results of binding activity of anti-HER3 antibody-drug conjugate KA0013 for surface HER3 of SK-BR-3 cells expressing HER3 on the cell surface.

FIG. 2: Assay results of internalization activity of anti-HER3 antibody-drug conjugate KA0013 in MDA-MB-453 cells expressing HER3 on the cell surface.

FIG. 3: Assay results of stability for non-specific killing caused by the cleavage of the linker-payload moiety of anti-HER3 antibody-drug conjugate KA0013.

FIG. 4: Assay results of plasma stability of anti-HER3 antibody-drug conjugates KA0013 and KA0011.

FIG. 5: *In vitro* assay of the cleavage efficiency of the linker-payload moieties of KA0013 and KA0011 mediated by cathepsin B.

FIG. 6: Assay results of binding activity of anti-HER3 antibody-drug conjugates KA0013 and KA0011 for antigen human HER3-his by indirect ELISA.

FIG. 7: Assay results of kinetic characteristic parameters of anti-HER3 antibody-drug conjugate KA0013.

FIG. 8: Assay results of kinetic characteristic parameters of anti-HER3 antibody-drug conjugate KA0011.

FIG. 9: Assay results of specific cell killing activity of anti-HER3 antibody-drug conjugates.

FIG. 10: Assay results of ADCC activity of anti-HER3 antibody-drug conjugates.

FIG. 11: *In vivo* efficacy results of anti-HER3 antibody-drug conjugates in CB-17 SCID mouse model of HCC827 human lung cancer cell xenograft tumor.

FIG. 12: Effect of anti-HER3 antibody-drug conjugates on body weight of CB-17 SCID mouse model of HCC827 human lung cancer cell xenograft tumor.

DETAILED DESCRIPTION

Embodiments of the present disclosure will be described in detail below with reference to examples, but those skilled in the art will understand that the following examples are only for illustrating the present disclosure and should not be construed as limitations to the scope of the present disclosure. The experimental procedures without specified conditions in the examples are conducted under conventional conditions or conditions recommended by the manufacturer. The used reagents or instruments without specified manufacturers are all commercially available conventional products.

In the following experimental examples of the present disclosure, the positive control ADC drug KA0011 used is the Her3 antibody-conjugate drug U3-1402 developed by Daiichi Sankyo Pharmaceutical Co., Ltd., Japan. For the sequence information and preparation method therefor, refer to Chinese Patent No. CN 106163559 B.

In the following experimental examples of the present disclosure, the isotype control antibodies used, i.e., the IgG (i.e., anti-HEL) components in IgG-Dxd, IgG-KA0012, and IgG-KA0010, and hIgG1 and anti-HEL, are all antibodies targeting human anti-hen egg lysozyme (HEL). The variable region sequences of these antibodies are derived from the study reported by Acierno et al., entitled "Affinity maturation increases the stability and plasticity of the Fv domain of anti-protein antibodies" (Acierno et al., J Mol Biol., 2007; 374(1): 130-46). For the constant region fragments, an Ig gamma-1 chain C region (ACCESSION: P01857) is used as the heavy chain constant region and an Ig kappa chain C region (ACCESSION: P01834) is used as the light chain constant region. IgG-DXd or IgG-KA0012, hIgG1, and anti-HEL are all prepared in the laboratory of Akeso Biopharma Inc.

<u>Preparation Example 1: Preparation of Anti-HER3 Antibody Patritumab</u>

Amino acid sequence of the heavy chain variable region of patritumab:

QVQLQQWGAGLLKPSETLSLTCAVYGGSFSGYYWSWIRQPPGKGLEWIGEINHSGSTNYNPSLKSRVTISVETSKNQFSLKLSSVTAADTAVYYCARDKWTWYFDLWGRGTLVTVSS (SEQ ID NO: 7)

Nucleotide sequence encoding the heavy chain variable region of patritumab:

CAGGTGCAGCTGCAGCAGTGGGGCGCCGGACTGCTGAAGCCATCCGAGACCCTGTCTCTGACATGTGCCGTGTACGGCGGCTCCTTTTCTGGCTACTATTGGAGCTGGATCAGGCAGCCCCCTGGCAAGGGACTGGAGTGGATCGGCGAGATCAACCACAGCGGCTCCACCAACTATAATCCCTCTCTGAAGAGCCGGGTGACCATCAGCGTGGAGACATCCAAGAATCAGTTCTCCCTGAAGCTGAGCTCCGTGACCGCAGCAGACACAGCCGTGTACTATTGCGCCCGGGACAAGTGGACCTGGTACTTTGATCTGTGGGGCAGAGGCACCCTGGTGACAGTGTCTAGC (SEQ ID NO: 8)

Amino acid sequence of the light chain variable region of patritumab:

DIEMTQSPDSLAVSLGERATINCRSSQSVLYSSSNRNYLAWYQQNPGQPPKLLIYWASTRESGVPDRFSGSGSGTDFTLTISSLQAEDVAVYYCQQYYSTPRTFGQGTKVEIK (SEQ ID NO: 9)

Nucleotide sequence encoding the light chain variable region of patritumab:

GACATCGAGATGACCCAGTCCCCTGATTCTCTGGCCGTGAGCCTGGGAGAGAGGGCAACAATCAACTGTAGAAGCTCCCAGTCCGTGCTGTACTCTAGCTCCAACCGGAATTACCTGGCCTGGTATCAGCAGAATCCAGGCCAGCCCCCTAAGCTGCTGATCTATTGGGCCAGCACCAGGGAGTCCGGAGTGCCAGACCGCTTCTCCGGCTCTGGCAGCGGCACAGACTTCACCCTGACAATCTCTAGCCTGCAGGCCGAGGACGTGGCCGTGTACTATTGCCAGCAGTACTATAGCACCCCCAGGACATTTGGCCAGGGCACCAAGGTGGAGATCAAG (SEQ ID NO: 10)

The heavy chain constant region of patritumab was an Ig gamma-1 chain C region, and the light chain constant region was an Ig kappa chain C region.

Amino acid sequence of the heavy chain constant region of patritumab:

ASTKGPSVFPLAPSSKSTSGGTAALGCLVKDYFPEPVTVSWNSGALTS

GVHTFPAVLQSSGLYSLSSVVTVPSSSLGTQTYICNVNHKPSNTKVDK
RVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTV
LHQDWLNGKEYKCKVSNKALPAPIEKTISKAKGQPREPQVYTLPPSRE
EMTKNQVSLTCLVKGFYPSDIAVEWESNGQPENNYKTTPPVLDSDGS
FFLYSKLTVDKSRWQQGNVFSCSVMHEALHNHYTQKSLSLSPGK
(SEQ ID NO: 11)

Nucleotide sequence encoding the heavy chain constant region of patritumab:

GCCTCTACAAAGGGGCCCAGCGTGTTTCCTCTGGCCCCTAGCAGCA
AGTCTACAAGCGGAGGAACAGCAGCTCTGGGCTGCCTGGTGAAGG
ACTACTTCCCAGAGCCCGTGACCGTGTCTTGGAACTCAGGAGCCCT
GACCAGCGGAGTGCACACATTTCCAGCCGTGCTGCAGAGCAGCGG
ACTGTATAGCCTGAGCAGCGTGGTGACCGTGCCTTCTTCTAGCCTG
GGCACCCAGACCTACATCTGCAACGTGAACCACAAGCCCAGCAAC
ACCAAGGTGGACAAGAGGGTGGAGCCCAAGTCTTGCGACAAGACC
CACACTTGTCCTCCTTGTCCAGCCCCAGAGCTGCTGGGAGGACCAA
GCGTGTTCCTGTTCCCTCCCAAGCCCAAGGACACCCTGATGATCAG
CAGGACCCCAGAAGTGACTTGCGTGGTGGTGGACGTGTCTCACGA
GGACCCCGAAGTGAAGTTCAATTGGTACGTGGACGGCGTGGAGGT
GCACAACGCTAAGACCAAGCCCAGGGAGGAGCAGTACAACAGCA
CCTACCGGGTGGTGTCCGTGCTGACAGTGCTGCACCAGGATTGGCT
GAACGGCAAGGAGTACAAGTGCAAGGTGTCCAATAAGGCCCTGCC
AGCCCCTATCGAGAAGACCATCAGCAAGGCCAAGGGCCAGCCTAG
AGAGCCTCAGGTGTACACCCTGCCTCCTTCTCGGGAGGAGATGAC
CAAGAACCAGGTGTCCCTGACTTGCCTCGTGAAGGGCTTCTACCCC
AGCGATATTGCCGTCGAGTGGGAGTCTAACGGCCAGCCCGAGAAC
AACTACAAGACCACACCTCCAGTGCTGGATAGCGACGGCAGCTTC
TTCCTGTACAGCAAGCTGACCGTGGACAAAAGCCGCTGGCAGCAG
GGCAACGTGTTTTCTTGCAGCGTGATGCACGAAGCCCTGCACAACC

ACTACACCCAGAAGAGCCTGAGCCTGTCTCCAGGCAAG (SEQ ID NO: 12)

Amino acid sequence of the light chain constant region of patritumab:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 13)

Nucleotide sequence encoding the light chain constant region of patritumab:

CGTACGGTGGCAGCCCCATCTGTCTTCATTTTCCCCCTAGTGACGAGCAGCTGAAATCCGGAACAGCCTCTGTGGTCTGTCTGCTGAACAATTTCTACCCTCGCGAAGCCAAGGTGCAGTGGAAAGTCGATAACGCTCTGCAGAGTGGCAATTCACAGGAGAGCGTGACTGAACAGGACTCCAAGGATTCTACCTATAGTCTGAGCTCCACTCTGACCCTGTCCAAAGCAGATTACGAAAAGCACAAAGTGTATGCCTGTGAGGTCACCCACCAGGGGCTGAGTTCTCCAGTCACCAAATCCTTCAACAGAGGCGAATGT (SEQ ID NO: 14)

The heavy chain cDNA and light chain cDNA of patritumab were separately cloned into a pcDNA3.1 vector to obtain a recombinant expression plasmid of the antibody patritumab. The recombinant plasmid was transfected into 293F cells. The 293F cell culture was purified and then detected.

The anti-HER3 monoclonal antibody patritumab was thus obtained.

According to the IMGT numbering system, six CDRs of the anti-HER3 monoclonal antibody patritumab are as follows:

HCDR1: GGSFSGYY (SEQ ID NO: 1)
HCDR2: INHSGST (SEQ ID NO: 2)
HCDR3: ARDKWTWYFDL (SEQ ID NO: 3)
LCDR1: QSVLYSSSNRNY (SEQ ID NO: 4)
LCDR2: WAS (SEQ ID NO: 5)
LCDR3: QQYYSTPRT (SEQ ID NO: 6)

Preparation Example 2: Preparation of Mc-AAA-Dxd (KA0012)

Mc-AAA-Dxd is (maleimide-N-yl)-$CH_2CH_2CH_2CH_2CH_2$-C(=O)-AAA-NH-$CH_2$-O-$CH_2$-C(=O)-(NH-DX). The CAS number of Dxd is: 1599440-33-1. Mc-AAA-Dxd is named KA0012 in the present disclosure, the structure of which is represented by Formula II below:

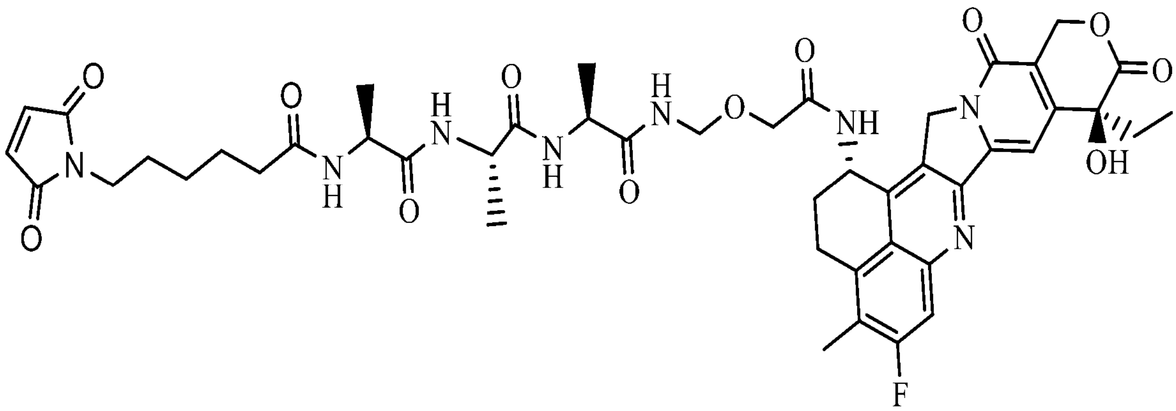

Formula II

wherein Mc is maleimide-N-yl, the structure of which is represented by Formula III below:

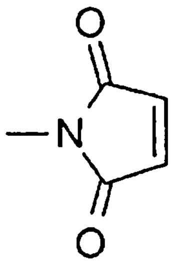

Formula III

wherein the structural formula of -(NH-DX) is represented by Formula IV below:

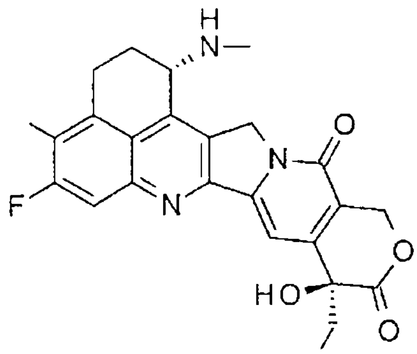

Formula IV

The synthetic steps of KA0012 are shown as step 1 to step 10 below.

Step 1: Preparation of intermediate HM-1315 6B 2

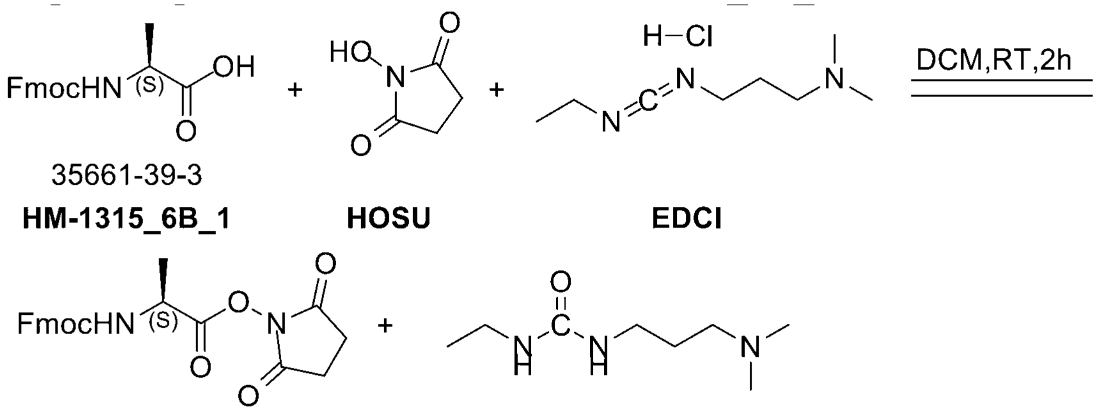


35661-39-3

**HM-1315_6B_1** **HOSU** **EDCI**

**HM-1315_6B_2**

The starting material HM-1315_6B_1 (450.0 g, 1.44 mol, 1 eq) was suspended in DCM (dichloromethane) (4.5 L, 10 V), and HOSU (182.9 g, 1.59 mol, 1.1 eq) and EDCI (304.8 g, 1.59 mol, 1.1 eq) were sequentially added with the temperature controlled at 0-5 °C. After the addition, the reaction mixture was dissolved until a clear solution was obtained. The solution was reacted at 10-20 °C for 3 h. Semi-saturated brine (1.0 L) was added to the reaction mixture, and the resulting mixture was stirred and left to stand for liquid separation. Then the organic phase was washed once with semi-saturated brine (1.0 L), washed twice with saturated brine (1.0 L × 2), dried over anhydrous sodium sulfate, and concentrated to give a white solid (579.0 g, yield: 98%).

$^1$H NMR (400 MHz, DMSO) δ 8.13 (d, J = 7.4 Hz, 1H), 7.89 (d, J = 7.5 Hz, 2H), 7.71 (t, J = 6.7 Hz, 2H), 7.42 (t, J = 7.3 Hz, 2H), 7.33 (t, J = 7.4 Hz, 2H), 4.52 (t, J=7.4 Hz, 1H), 4.41-4.17 (m, 3H), 2.79 (d, J=15.5 Hz, 4H), 2.59 (s, 1H), 1.46 (d, J =7.4 Hz, 3H). LCMS [M+23]: 431.1.

Step 2: Preparation of intermediate HM-1315_1

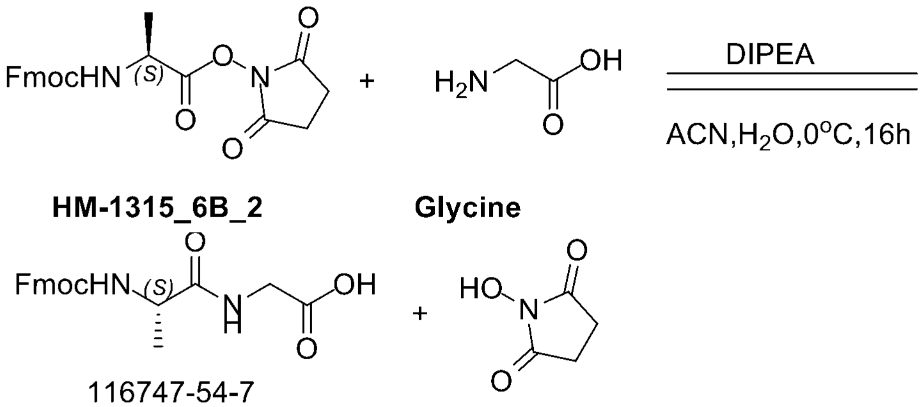


**HM-1315_6B_2** **Glycine**

116747-54-7

**HM-1315_1**

The starting material HM-1315_6B_2 (579.0 g, 1.42 mol, 1.0 eq) was dissolved in acetonitrile (2895 mL, 5 v), and glycine (117.2 g, 1.56 mol, 1.1 eq) and water (2895 mL, 5 v) were added. The mixture was cooled to -5-0 °C, and DIPEA (N,N-diisopropylethylamine) (275.3 g, 2.13 mol, 1.5 eq) was slowly added. After the addition, the mixture was reacted at 20-25 °C for 3 h. The reaction mixture was diluted with ethyl acetate (1.0 L) and adjusted to pH 3-4 with diluted hydrochloric acid (0.5 N). Ethyl acetate (500 mL) was added for extraction and liquid separation. The aqueous phase was extracted twice with ethyl acetate (1.0 L × 2). The organic phases were combined, washed once with saturated brine (1.5 L), dried over anhydrous sodium sulfate, filtered, and concentrated. The crude product was slurried with methyl tert-butyl ether/ethyl acetate (2895 mL/289.5 mL, 5 v/0.5 v) for 2 h and filtered, and the filter cake was dried with an oil pump to give a white powdery solid (447.2 g, yield: 85%). $^1$H NMR (400 MHz, DMSO) δ 8.12 (t, J=5.5 Hz, 1H), 7.89 (d, J = 7.5 Hz, 2H), 7.73 (t, J = 7.1 Hz, 2H), 7.54 (d, J=7.8 Hz, 1H), 7.37 (dt, J = 34.6, 7.1 Hz, 4H), 4.24 (dd, J=15.6, 5.9 Hz, 3H), 4.13- 4.05 (m, 1H), 3.75 (dd, J=14.9, 5.7 Hz, 3H), 1.23 (d, J=7.2 Hz, 3H). LCMS [M+1]: 369.1, [M+23]: 392.1.

Step 3: Preparation of intermediate HM-1315_2

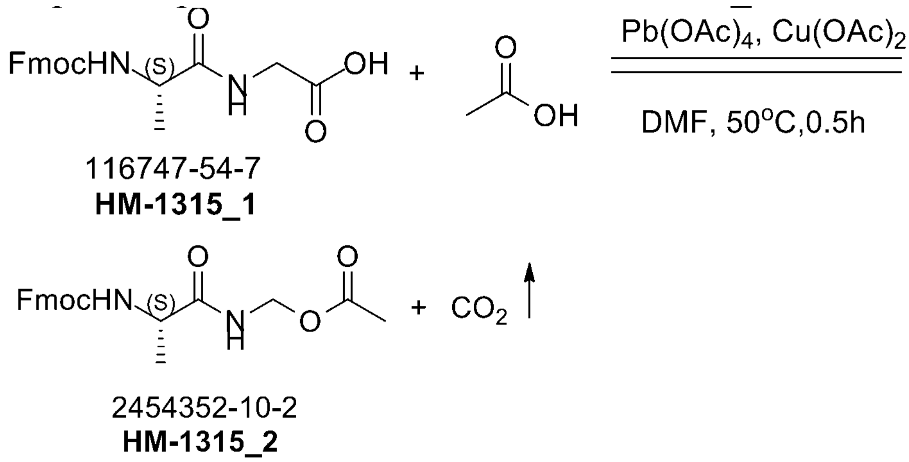


116747-54-7
**HM-1315_1**

2454352-10-2
**HM-1315_2**

The starting material HM-1315_1 (474.0 g, 1.29 mol, 1.0 eq) was dissolved in ultra-dry DMF (2370 mL, 5 v). Under nitrogen atmosphere, lead tetraacetate (1143.9 g, 2.58 mol, 2.0 eq), anhydrous copper acetate (234.3 g, 1.29 mol, 1.0 eq), and acetic acid (170.5 g, 2.84 mol, 2.2 eq) were added. After the addition, the mixture was heated to about 50 °C. The reaction mixture was reacted under an oil bath at 50-55 °C for 0.5 h. The reaction mixture was cooled, and ethyl acetate (2370 mL, 5 v) was added, followed by the addition of ice water (2370 mL, 5 v). A black viscous solid was precipitated. The reaction mixture was filtered through a Buchner funnel to remove black insoluble substances, and the filtrate was subjected to liquid separation using a separatory funnel. The aqueous layer was extracted twice with ethyl acetate (2.0 L × 2). The organic phases were combined, washed twice with water (1.0 L × 2), washed three times with saturated brine (1.0 L × 3), dried over anhydrous sodium sulfate, filtered, and concentrated. The resulting crude product was slurried with methyl tert-butyl ether (2370 mL) at room temperature for 2 h and filtered, and the filter cake was dried with an oil pump to give a white solid product (367.7 g, yield: 74%).

$^1H$ NMR (400 MHz, DMSO) δ 8.90 (s, 1H), 7.89 (d, J =7.5 Hz, 2H), 7.73 (t, J=6.6 Hz, 2H), 7.59 (d, J=7.6 Hz, 1H), 7.42 (t, J =7.4 Hz, 2H), 7.33 (t, J=7.4 Hz, 2H), 5.10 (d, J=6.7 Hz, 2H), 4.25 (dd, J=20.3, 6.5 Hz, 3H), 4.12-4.01 (m, 1H), 1.99 (s, 3H), 1.22 (d, J=7.2 Hz, 3H). LCMS [M+23]: 405.0.

Step 4: Preparation of intermediate HM-1315 4

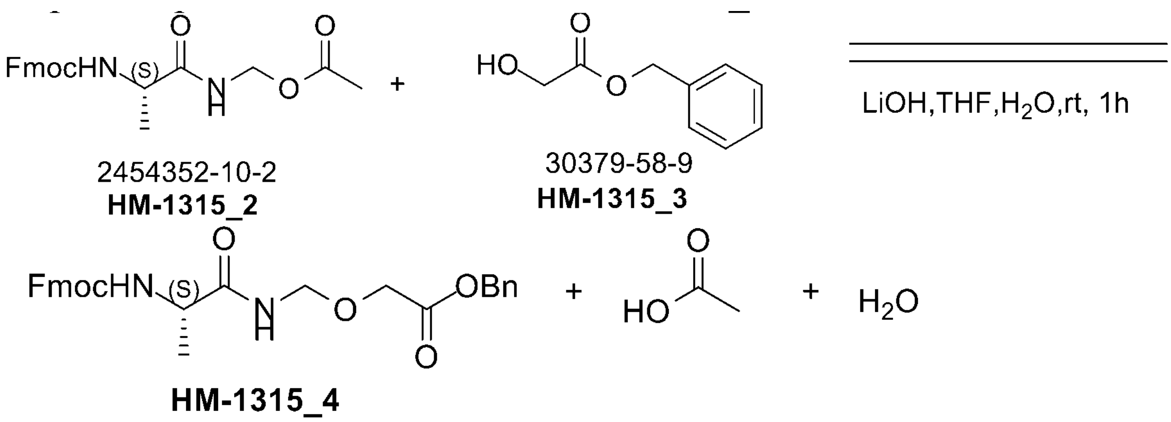


2454352-10-2
**HM-1315_2**

30379-58-9
**HM-1315_3**

**HM-1315_4**

The starting material HM-1315_2 (326.0 g, 0.85 mol, 1.0 eq) was dissolved in THF (3260.0 mL, 10 v), and HM-1315_3 (211.9 g, 1.27 mol, 1.5 eq) was added. After the addition, the reaction was cooled to 0-10 °C, and an aqueous solution (163.0 mL, 0.5 v) of lithium hydroxide (24.4 g, 1.02 mol, 1.2 eq) was slowly added. After the addition, the mixture was naturally warmed to room temperature (15-25 °C) and reacted for 1 h. Ethyl acetate (1.5 L) and water (1.5 L) were added to the reaction mixture, followed by liquid separation. The aqueous layer was extracted once with ethyl acetate (1.0

L). The organic phases were combined, washed twice with saturated brine (1.0 L × 2), dried over anhydrous sodium sulfate, filtered, and concentrated. The resulting crude product was purified by column chromatography (elution: petroleum ether/ethyl acetate = 1/1). The product was collected and concentrated, and the residue was dried with an oil pump to give a white solid product (311.8 g, yield: 74%).

$^1$H NMR (400 MHz, DMSO) δ 8.74 (s, 1H), 7.89 (d, J=7.5 Hz, 2H), 7.72 (t, J=7.9 Hz, 2H), 7.59 (d, J=7.3 Hz, 1H), 7.47-7.25 (m, 9H), 5.13 (s, 2H), 4.64 (d, J=6.7 Hz, 2H), 4.31-4.18 (m, 3H), 4.15 (s, 2H), 4.07-3.95 (m, 1H), 1.23 (d, J=7.2 Hz, 3H). LCMS [M+23]: 511.1.

Step 5: Preparation of intermediate HM-1315_7

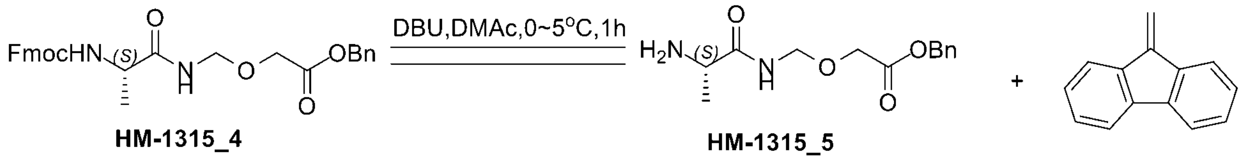


HM-1315_4

HM-1315_5

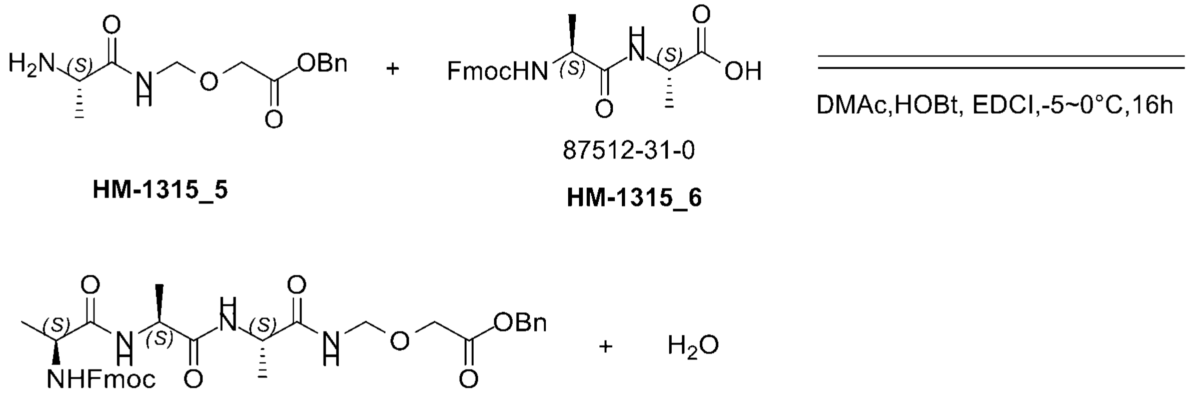


HM-1315_5

87512-31-0

HM-1315_6

2414594-25-3

HM-1315_7

The starting material HM-1315_4 (60.00 g, 122.82 mmol, 1.0 eq) was dissolved in DMAc (N,N-dimethylacetamide) (600.0 mL, 10 v). The solution was cooled to 0-5 °C, and DBU (1,8-diazabicyclo[5.4.0]undec-7-ene) (18.70 g, 122.82 mmol, 1.0 eq) was slowly added. After the addition, the mixture was reacted at 0-5 °C for 1 h. The reaction mixture was cooled to -5-0 °C, and PPTS (pyridinium 4-methylbenzenesulfonate) (30.86 g, 122.82 mmol, 1.0 eq), EDCI (23.54 g, 122.82 mmol, 1.0 eq), HOBT (1-hydroxybenzotriazole) (16.59 g, 122.82 mmol, 1.0 eq), and HM-1315_6 (42.27 g, 110.54 mmol, 0.9 eq) were sequentially added to the reaction mixture. After the addition, the mixture was reacted at -5-0 °C for 16 h. n-Hexane (1200 mL, 20 v) was added to the reaction mixture, and the resulting mixture was left to stand for liquid separation. The DMAc layer was washed four times with n-hexane (1.2 L × 4) and then poured into water (1800 mL, 30 v), and a large amount of white solid precipitated. The mixture was filtered through a Buchner funnel, and the filter cake was dried to give a crude product as a white solid. Acetonitrile (300.0 mL, 5 v) was added to a reaction flask, and the white crude product obtained in the previous step was added. The mixture was slurried for 1 h and filtered, and the filter cake was dried with an oil pump to give a white solid product (56.2 g, yield: 80%).

$^1$H NMR (400 MHz, DMSO) δ 8.68 (t, J = 6.7 Hz, 1H), 7.99 (dd, J = 15.4, 7.2 Hz, 2H), 7.89 (d, J=7.5 Hz, 2H), 7.72 (t, J=7.0 Hz, 2H), 7.54 (d, J=7.4 Hz, 1H), 7.46-7.28 (m, 9H), 5.14 (s, 2H), 4.63 (d, J = 7.7 Hz, 2H), 4.32-4.16 (m, 5H), 4.13 (s, 2H), 4.09-4.01 (m, 1H), 1.21 (dd, J = 7.0, 2.6 Hz, 9H). LCMS [M+23]: 654.3.

Step 6: Preparation of intermediate HM-1315_8

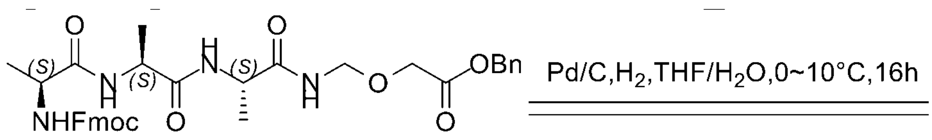


2414594-25-3

**HM-1315_7**

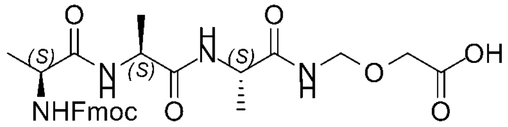

+

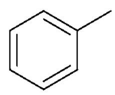

**HM-1315_7_2**

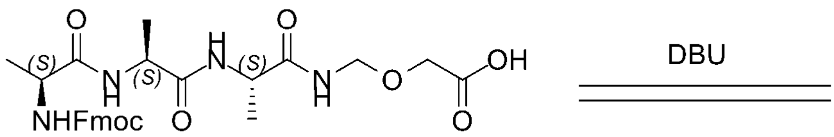


**HM-1315_7_2**

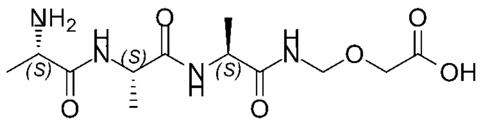

+

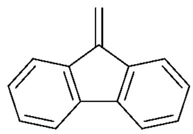

2414594-27-5

**HM-1315_8**

The starting material HM-1315_7 (2.59 g, 4.10 mmol, 1.0 eq) was suspended in THF (tetrahydrofuran) (51.8 mL, 20 v) and water (5.2 mL, 2 v). The suspension was cooled to 0-10 °C, and 10% palladium on carbon (0.39 g, 0.15 w/w) was added. The mixture was purged three times with hydrogen and reacted at 0-10 °C for 16 h. The reaction mixture was filtered to remove palladium on carbon, and the filter cake was rinsed with acetonitrile/water (10 mL/10 mL). The filtrate was extracted with 2-methyltetrahydrofuran (50 mL). The organic phase was washed once with water (50 mL) and saturated brine (50 mL), dried over anhydrous sodium sulfate, filtered, and concentrated to give a crude product as a white solid (2.0 g). The crude product (2.0 g, 3.70 mmol, 1.0 eq) was suspended in dichloromethane (20.0 mL, 10 v), and DBU (0.56 g, 3.70 mmol, 1.0 eq) was slowly added dropwise with the temperature controlled at 20-25 °C. After the addition, the reaction mixture was partially dissolved. The mixture was reacted at 20-25 °C for 16 h, during which a white solid was precipitated. Methyl tert-butyl ether (40.0 mL, 20 v) was added to the reaction mixture, and a greater amount of white solid was precipitated. The mixture was filtered, and the filter cake was transferred to a single-necked flask. Acetonitrile (20.0 mL) was added, and the mixture was slurried for 2 h and filtered. The filter cake was dried with an oil pump to give a white solid product (0.5 g, yield: 38%).

$^1$H NMR (400 MHz, D2O) δ 4.73 (q, J=10.8 Hz, 1H), 4.43- 4.21 (m, 1H), 4.04-3.82 (m, 1H), 1.47 (d, J=7.0 Hz, 2H), 1.42 (d, J=7.2 Hz, 3H). LCMS [M+1]: 319.1.

Step 7: Preparation of intermediate HM-1315_9

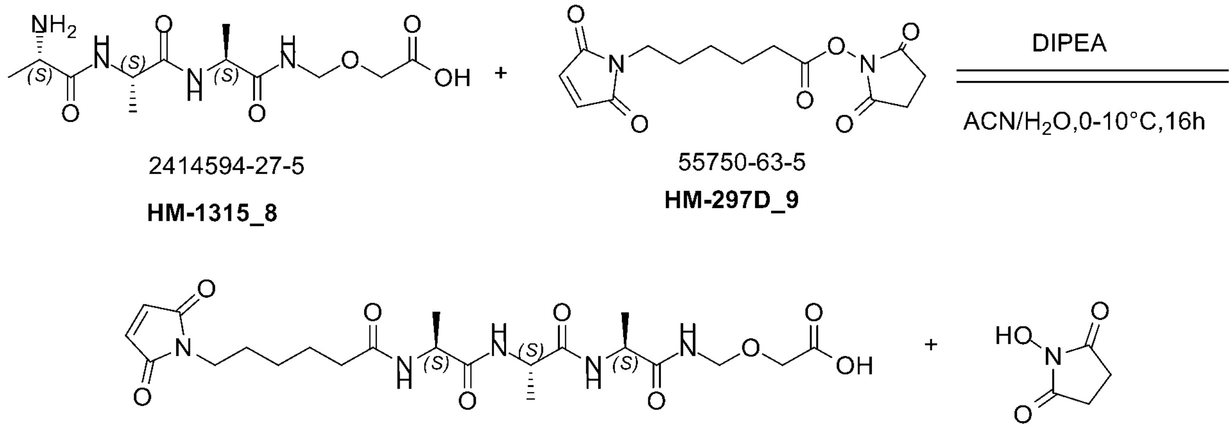


2414594-27-5

**HM-1315_8**

55750-63-5

**HM-297D_9**

**HM-1315_9**

The starting material HM-1315_8 (300 mg, 0.94 mmol, 1.0 eq) was dissolved in acetonitrile (3.0 mL, 10 v) and water (4.5 mL, 15 v), and HM-297D_9 (290 mg, 0.94 mmol, 1.0 eq) was added. The mixture was cooled to 0-10 °C, and DIPEA (121 mg, 0.94 mmol, 1.0 eq) was added. The mixture was reacted at 0-10 °C for 16 h and then separated by preparative chromatography to give a product (290 mg, yield: 60%) as a white solid.

$^1H$ NMR (400 MHz, DMSO) δ 8.61 (t, J=6.7 Hz, 1H), 7.97 (dd, J = 7.1, 3.4 Hz, 2H), 7.91 (d, J=7.3 Hz, 1H), 7.00 (s, 2H), 4.66-4.52 (m, 2H), 4.29-4.12 (m, 3H), 3.95 (s, 2H), 3.44-3.35 (m, 5H), 2.08 (t, J=7.4 Hz, 2H), 1.54-1.40 (m, 4H), 1.28-1.10 (m, 11H). LCMS [M+23]: 534.3.

Step 8: Preparation of intermediate HM-582_9

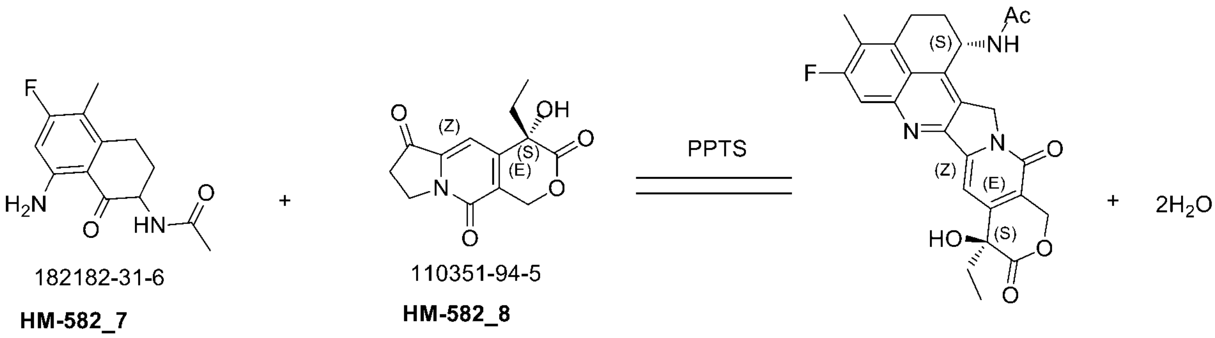


182182-31-6

**HM-582_7**

110351-94-5

**HM-582_8**

**HM-582_9**

Compound HM-582_7 (10 g, 39.98 mmol, 1.0 eq), compound HM-582_8 (10 g, 38.01 mmol, 0.95 eq), and pyridinium p-toluenesulfonate (6 g, 23.88 mmol, 0.60 eq) were added to a three-necked flask, and toluene (500 mL) was added. The mixture was reacted at 130-140°C with water separation for 48 h. The reaction mixture was cooled, filtered, and washed with methyl tert-butyl ether (100 mL). The solid was collected and dried to give the product (18.4 g, 96.5%).

$^1H$ NMR (DMSO) δ: 8.43-8.55 (m, 1H), 7.80 (d, J = 10.9 Hz, 1H), 7.31 (d, J = 3.8 Hz, 1H), 6.55 (brs, 1H), 5.52-5.57 (m, 1H), 5.42 (s, 2H), 5.16-5.20 (m, 2H), 3.14-3.18 (m, 2H), 2.39 (s, 3H), 2.11-2.16 (m, 2H), 1.80-1.92 (m, 5H), 0.86-0.89 (m, 3H). LCMS [M+1] 478.2.

Step 9: Preparation of intermediate HM-582_10

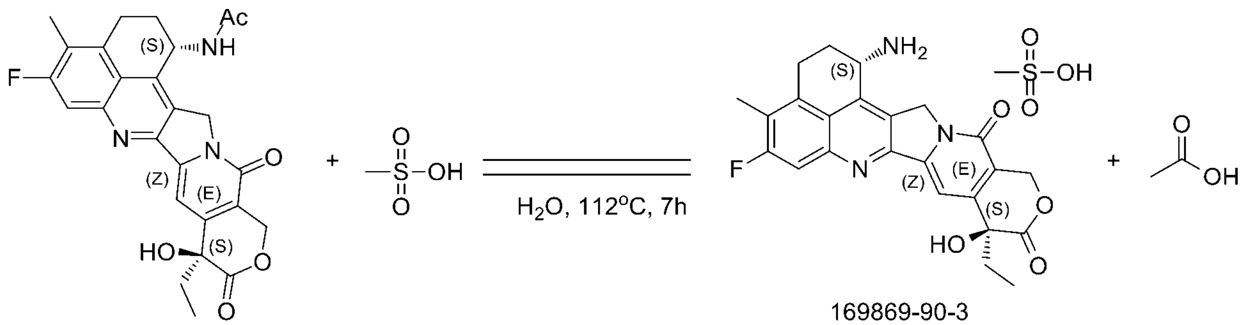


169869-90-3

**HM-582_10**

**HM-582_9**

Compound HM-582_9 (30 g) was suspended in water (600 mL), and methanesulfonic acid (300 mL) was slowly added. The solid was dissolved with an exothermic effect. After purging with nitrogen, the mixture was heated to 112 °C and reacted for 7 h. The reaction mixture was cooled to room temperature and filtered, and the filter cake was washed with water (100 mL). The filtrate was diluted with ethanol (4 L), and a solid was precipitated. The mixture was stirred at room temperature for 20 min, filtered, and dried under reduced pressure. The crude product was suspended in ethanol/water (4:1, 1 L), heated at reflux for 2 h, cooled to room temperature, and filtered. The solid was washed with a small amount of ethanol, dried under reduced pressure, and lyophilized to obtain exatecan mesylate (16.7 g, 50%).

$^1$H NMR (DMSO) δ 8.41-8.50 (m, 3H), 7.86 (d, J = 10.8 Hz, 1H), 7.33 (s, 1H), 5.66-5.74 (m, 1H), 5.37-5.44 (m, 2H), 5.10 (s, 1H), 3.27-3.32 (m, 1H), 3.08-3.17 (m, 1H), 2.41 (s, 3H), 2.31 (s, 3H), 2.17-2.24 (m, 1H), 1.80-1.95 (m, 2H), 0.88 (t, J = 7.3 Hz, 3H). LCMS [M+1] 436.1.

Step 10: Preparation of HM-1315_10 (KA0012)

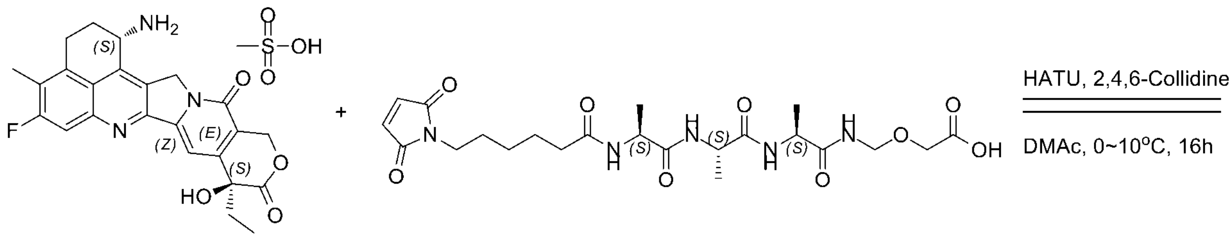


169869-90-3

**HM-582_10**

2414594-28-6

**HM-1315_9**

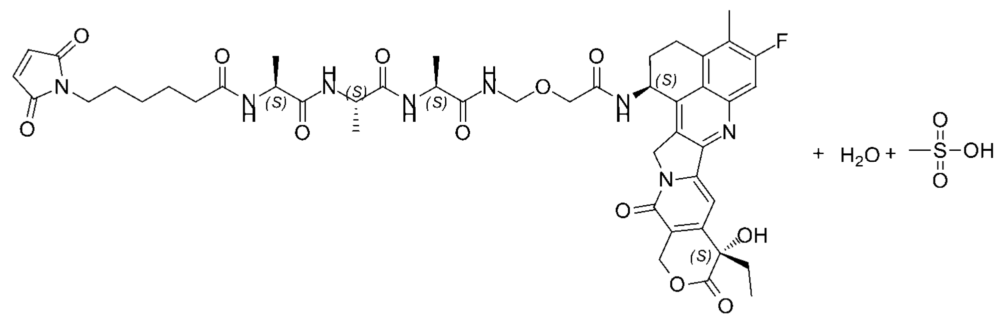

***HM-1315_10***

The starting material HM-1315_9 (150 mg, 0.29 mmol, 1.0 eq) was dissolved in DMAc (3.0 mL, 20 v), and HM-582_10 (156 mg, 0.29 mmol, 1.0 eq) was added. The mixture was cooled to 0-10 °C, and HATU (2-(7-azabenzotriazol-1-yl)-N,N,N',N'-tetramethyluronium hexafluorophosphate) (121 mg, 0.32 mmol, 1.0 eq) was added, followed by dropwise addition of 2,4,6-trimethylpyridine (70 mg, 0.58 mmol, 2.0 eq). The mixture was reacted at 0-10 °C for 16 h. After

the starting material was completely converted as shown by LCMS, the mixture was separated by preparative chromatography to give a product (135 mg, yield: 50%) as a pale yellow solid product.

$^1H$ NMR (400 MHz, DMSO) δ 8.64 (t, J = 6.6 Hz, 1H), 8.47 (d, J = 8.8 Hz, 1H), 7.95 (dd, J = 9.3, 7.3 Hz, 2H), 7.87 (d, J = 7.2 Hz, 1H), 7.77 (d, J = 11.0 Hz, 1H), 7.30 (s, 1H), 6.99 (s, 2H), 6.51 (s, 1H), 5.60 (d, J = 8.0 Hz, 1H), 5.42 (s, 2H), 5.18 (s, 2H), 4.61 (dt, J = 10.1, 3.3 Hz, 2H), 4.17 (dt, J = 14.1, 7.1 Hz, 3H), 3.98 (s, 2H), 3.38 (s, 1H), 3.16 (dd, J = 14.1, 6.4 Hz, 2H), 2.38 (s, 3H), 2.18 (d, J = 5.4 Hz, 2H), 2.07 (t, J = 7.3 Hz, 2H), 1.85 (dd, J = 15.0, 7.4 Hz, 2H), 1.51 - 1.39 (m, 4H), 1.17 (dt, J = 14.5, 7.4 Hz, 11H), 0.86 (t, J = 7.3 Hz, 3H). LCMS [M+1]:929.3, [M+23]:952.3.

Preparation Example 3: Preparation of Anti-HER3 Antibody-Drug Conjugate KA0013

The structures of KA0013 and KA0011 are shown in Table 1 below.

Table 1

| Name | Structure |
|---|---|
| KA0013 | Patritumab -Mc-AAA-Dxd |
| KA0011 | Patritumab -Mc-GGFG-Dxd |

An antibody-drug conjugate is obtained by forming a thioether bond in a disulfide bond site present in a hinge portion of an anti-HER3 antibody, wherein the anti-HER3 antibody is treated under reducing conditions and then reacted with a compound (such as Formula II) to give the antibody-drug conjugate. Specifically, an antibody-drug conjugate can be produced by reacting the compound with an antibody having a sulfhydryl group.

Antibodies having a sulfhydryl group can be obtained using methods well known in the art (Hermanson, G. T, Bioconjugate Techniques, pp. 56-136, pp. 456-493, Academic Press (1996)). Examples include, but are not limited to: reacting Traut's reagent with an amino group of the antibody; reacting N-succinimidyl S-acetylthioalkanoate with an amino group of the antibody followed by reaction with hydroxylamine; reacting the antibody with N-succinimidyl 3-(pyridyldithio) propionate followed by reaction with a reducing agent; and reacting the antibody with a reducing agent such as dithiothreitol, 2-mercaptoethanol, and tris(2-carboxyethyl)phosphine hydrochloride (TCEP) to reduce the disulfide bond in the hinge portion within the antibody to form sulfhydryl.

Specifically, an antibody in which a disulfide bond in a hinge portion in the antibody is partially or completely reduced can be obtained by using 0.3 to 10 molar equivalents of TCEP as a reducing agent per disulfide bond in the hinge portion in the antibody and reacting the reducing agent with the antibody in a buffer containing a chelating agent. Examples of the chelating agent include ethylenediaminetetraacetic acid (EDTA) and diethylenetriaminepentaacetic acid (DTPA).

The chelating agent may be at a concentration of 1 mM to 20 mM. Examples of buffers that can be used include sodium phosphate, sodium borate, and sodium acetate solutions. Specifically, an antibody having a partially or completely reduced sulfhydryl group can be obtained by reacting the antibody with TCEP at 4-37 °C for 1-4 h.

Meanwhile, by performing a reaction of adding a sulfhydryl group to the drug-linker moiety, the drug-linker moiety can be conjugated via a thioether bond. Using 2-20 molar equivalents of the compound per antibody having a sulfhydryl group, antibody-drug conjugates in which 2-8 drug molecules are conjugated per antibody can be produced. Specifically, it is sufficient to add a solution in which the compound is dissolved in a buffer containing the antibody having a sulfhydryl group to carry out the reaction. Here, examples of buffers that can be used include sodium acetate solution, sodium phosphate, and sodium borate. The reaction is performed at pH 5-9, and more preferably, the reaction is performed at about pH 7. Examples of solvents for dissolving compound (1) include organic solvents such as dimethyl sulfoxide (DMSO), dimethylformamide (DMF), dimethylacetamide (DMA), and N-methyl-2-pyridone (NMP).

The reaction may be performed by adding an organic solvent solution in which the compound is dissolved to a buffer containing the antibody having a sulfhydryl group at 1-20% v/v. The reaction temperature is 0-37°C, more preferably 10-25°C, and the reaction time is 0.5-2 h.

After conjugation, the manufactured antibody-drug conjugate can be subjected to buffer exchange, determination of antibody concentration, and determination of the average number of conjugated drugs per antibody molecule, so as to identify the antibody-drug conjugate.

Common procedure A: determination of antibody concentration

The antibody concentration was determined using a UV detector (Nanodrop 1000, Thermo Fisher Scientific Inc.) according to the method specified by the manufacturer. In this case, the extinction coefficient at 280 nm could be deduced from the amino acid sequence of the antibody using a known calculation method (Protein Science, 1995, vol. 4, 2411-2423), and absorbance coefficients at 280 nm (1.3 mL mg-1cm-1 to 1.8 mL mg-1cm-1) that vary from antibody

to antibody were used. In the case of KA0013, the extinction coefficient at 280 nm of 1.768 mL mg-1cm-1 was used as a deduced value based on the amino acid sequence thereof.

Common procedure B: buffer exchange for antibody-drug conjugate

The antibody-drug conjugate was subjected to ultrafiltration concentration and buffer exchange using an ultrafiltration membrane (Millipore P3C030C00, 30 kDa) according to the method specified by the manufacturer, with a 10-fold buffer exchange ratio.

Common procedure C: determination of average number of conjugated drugs per antibody molecule in antibody-drug conjugate

Determination of average number of conjugated drugs per antibody molecule in antibody-drug conjugate using liquid chromatography-mass spectrometry: The sample was diluted to 2 mg/mL with water. 50 μL of the diluted sample was taken, and 1 μL of 0.5 M TCEP was added. The mixture was reacted at room temperature for 30 min before injection. Chromatographic conditions: column: MabPac RP (2.1 × 50 mm); mobile phase: mobile phase A: 0.1% aqueous formic acid solution; mobile phase B: a 0.1% formic acid in acetonitrile; sample injection volume: 2 μg; column temperature: 80 °C; and detection wavelength: 280 nm.

The liquid phase gradient is shown in Table 2 below.

Table 2

| Time (min) | Flow rate (mL/min) | %A | %B |
|---|---|---|---|
| 0.00 | 0.30 | 90.0 | 10.0 |
| 3.00 | 0.30 | 90.0 | 10.0 |
| 7.00 | 0.30 | 10.0 | 90.0 |
| 8.00 | 0.30 | 10.0 | 90.0 |
| 8.10 | 0.30 | 90.0 | 10.0 |
| 10.00 | 0.30 | 90.0 | 10.0 |

The DAR value was calculated according to the signal values of components.

$$DAR = 2 \times \left( \frac{1 \times LC_1}{LC_0 + LC_1} + \frac{1 \times HC_1 + 2 \times HC_2 + 3 \times HC_3}{HC_1 + HC_2 + HC_3} \right)$$

In the above formula, LCn represents the signal value of the light chain conjugated with n linker-payloads, and HCn represents the signal value of the heavy chain conjugated with n linker-payloads.

Specific conjugation examples were as follows.

Reduction of antibody: Patritumab was diluted to about 10 mg/mL with phosphate buffer PB/EDTA (15 mM $Na_2HPO_4$, 5 mM $NaH_2PO_4$, 5 mM EDTA-2Na, pH 7.0). The antibody solution (100 mL) was added to a beaker at room temperature, and then a 10 mM aqueous TCEP solution (0.68 mL; 10.0 equivalents relative to one antibody molecule) was added. The mixture was stirred using a magnetic stirrer and incubated at room temperature for 3 h to reduce the disulfide bond in the hinge portion of the antibody.

Conjugation of antibody to drug linker: A solution of 10 mM KA0012 in DMSO (0.748 mL; 11.0 equivalents relative to one antibody molecule) was slowly added to the mixture. The mixture was stirred at room temperature for 1 h to conjugate the drug linker with the antibody.

Purification: Ultrafiltration concentration and buffer exchange were performed using an ultrafiltration membrane. While unconjugated drug linkers and other low-molecular-weight reagents were removed, the buffer was exchanged for a formulation buffer, and the solution was further concentrated. The resulting purified solution was sterilized by filtration to give a solution containing the antibody-drug conjugate KA0013.

The antibody concentration was determined to be 23.64 mg/mL using common procedure A, and the average number (n) of conjugated drugs per antibody molecule in KA0013 was determined to be 7.87 using common procedure C.

Preparation Example 4: Preparation of Control Drug

KA0010: Mc-GGFG-Dxd as a control linker-payload.

The structural formula of control KA0010 is represented by Formula V below:

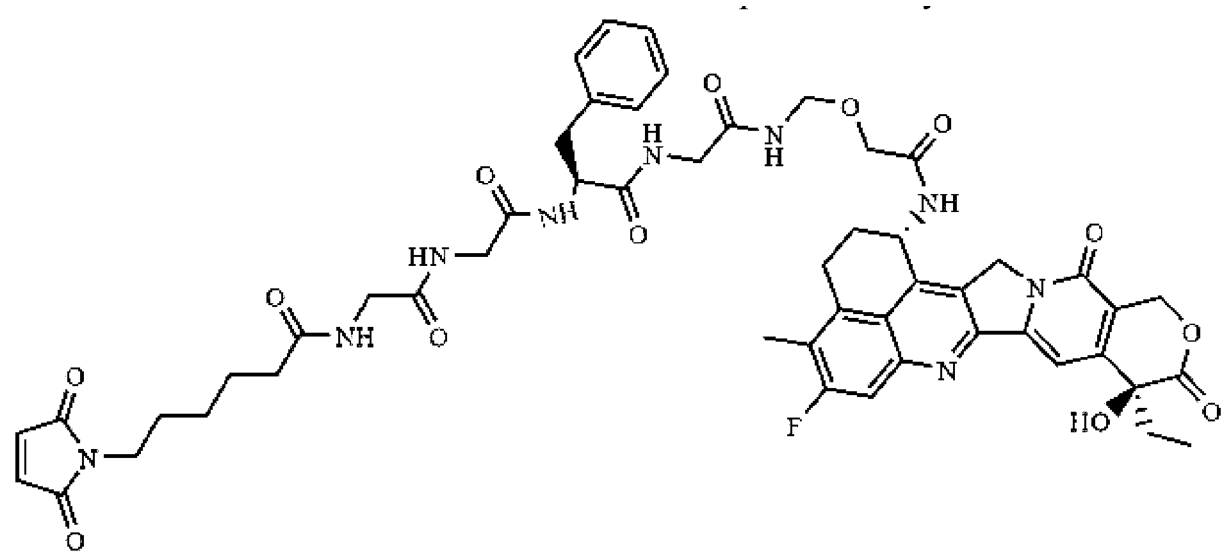

Formula V

KA0011: patritumab-Mc-GGFG-Dxd.
IgG-KA0010: IgG-Mc-GGFG-Dxd, abbreviated as IgG-Dxd.
IgG-KA0012: IgG-Mc-AAA-Dxd.

The preparation methods for KA0010 and KA0011 were performed by referring to the relevant descriptions in Chinese Patent No. CN 106163559 B. IgG-KA0010 and IgG-KA0012 were both prepared by the general conjugation method commonly used in sulfhydryl conjugation, and the specific preparation method was performed by referring to the above preparation method for KA0013, or by referring to the related descriptions in Chinese Patent No. CN 106163559 B.

Example 1: FACS Assay for Binding Activity of Anti-HER3 Antibody-Drug Conjugate KA0013 to HER3 on SK-BR-3 Cell Surface

1. Experimental drug

KA0013,
HER3-targeted monoclonal antibody patritumab (prepared according to Preparation Example 1).
KA0011.

2. Experimental method

Following standard procedures, SK-BR-3 cells (purchased from Shanghai Institutes for Biological Sciences, Chinese Academy of Sciences) were collected, washed once with an appropriate amount of PBS, counted, and determined for cell viability. The cell suspension was added to a 96-well plate at $3 \times 10^5$ cells/sample and centrifuged at 1000× g for 5 min. The supernatant was discarded. 100 μL of each of the serially diluted experimental drugs (900 nM, 300 nM, 100 nM, 33.3 nM, 11.1 nM, 3.7 nM, 1.23 nM, 0.41 nM, 0.041 nM, and 0.0041 nM based on antibody working concentrations, respectively) was added, and the cell pellet was resuspended and incubated on ice for 40 min. 1% PBSA (PBS + 1% BSA) was added, and the mixture was centrifuged at 1000× g for 5 min. The supernatant was discarded, and the cells were further washed twice. 100 μL of 300-fold diluted fluorescent antibody Mouse Anti-Human IgG Fc-Alexa Fluor 647 (Southern Biotech, Cat. No. 9040-31) was added, and the cell pellet was resuspended and incubated on ice for 30 min

in the dark. 1% PBSA was added, and the mixture was centrifuged at 1000× g for 5 min. The supernatant was discarded, and the cells were further washed twice. 200 μL of 1% PBSA was added, and the cell pellet was resuspended and then loaded into the flow cytometer for assay.

3. Experimental results

The results are shown in FIG. 1.

The results showed that under the same experimental conditions, the $EC_{50}$ values of KA0013, KA0011, and the HER3-targeted monoclonal antibody patritumab for binding to SK-BR-3 cells were 15.36 nM, 12.89 nM, and 8.481 nM, respectively.

The results indicate that under the same experimental conditions, KA0013, KA0011, and patritumab all have the activity of effectively binding to SK-BR-3 cells expressing HER3.

<u>Example 2: FACS Assay for Internalization Activity of Anti-HER3 Antibody-Drug Conjugate KA0013 in MDA-MB-453 Cells</u>

1. Experimental drug

KA0013,
IgG-Dxd,
KA0012,
KA0011.

2. Experimental method

MDA-MB-453 cells (purchased from Cobioer Biosciences Co., Ltd., Cat. No. CBP60386) were collected, counted, and determined for cell viability. The cell suspension was added to a 96-well low-adsorption plate at $1 \times 10^5$ cells/sample and centrifuged at 750× g for 5 min. The supernatant was discarded. 100 μL of the experimental drug (at a working concentration of 100 nM) was added, with an isotype control (IgG-Dxd, at a working concentration of 100 nM) designed in parallel. The mixture was mixed uniformly and incubated in an incubator at 37 °C for 1 h, 3 h, and 5 h. After the incubation was completed, 1% PBSA (PBS + 1% BSA) was added, and the mixture was centrifuged at 750× g for 5 min. The supernatant was discarded, and the cells were washed twice with 1% PBSA. 100 μL of patritumab (at a working concentration of 100 nM) was added, and the mixture was incubated on ice for 30 min. 1% PBSA (PBS + 1% BSA) was added, and the mixture was centrifuged at 750× g for 5 min. The supernatant was discarded, and the cells were further washed twice with 1% PBSA. 100 μL of 300-fold diluted fluorescent antibody Mouse Anti-Human IgG Fc-Alexa Fluor 647 (Southern Biotech, Cat. No. 9040-31) was added, and the cell pellet was resuspended and incubated on ice for 30 min in the dark. 1% PBSA (PBS + 1% BSA) was added, and the mixture was centrifuged at 1000× g for 5 min. The supernatant was discarded, and the cells were further washed twice. 200 μL of 1% PBSA was added, and the cell pellet was resuspended and then loaded into the flow cytometer for assay.

3. Experimental results

The results are shown in FIG. 2.

The results showed that under the same experimental conditions, KA0013 and KA0011 could be endocytosed in MDA-MB-453 as compared to isotype control IgG-Dxd (i.e., IgG-Mc-GGFG-Dxd, produced by Akeso Biopharma Inc.), and the endocytic activity of the two was significantly different from that of IgG-Dxd.

<u>Example 3: Determination of Non-Specific Killing Activity of Linker-Payload Moiety of Anti-HER3 Antibody-Drug Conjugates</u>

1. Experimental drug

IgG-KA0010,
IgG-KA0012.

2. Experimental method

The non-specific killing activity assay was performed on the linker-payload moiety Mc-AAA-Dxd (named KA0012) of the anti-HER3 antibody-drug conjugate KA0013 and the linker-payload moiety Mc-GGFG-Dxd (named KA0010) of the control KA0011.

After the human venous blood was collected, the heparin sodium anticoagulant tube was gently inverted for uniform mixing, and centrifuged at room temperature at 3500 rpm for 10 min. The plasma layer was collected for the experiment. MDA-MB-453 cells were routinely collected, counted, and determined for cell viability. The cell density was adjusted to $2.5 \times 10^4$ cells/mL, and the cell suspension was added to a 96-well black-bottom plate at 100 μL/well. The plate was incubated in an incubator at 37 °C with 5% $CO_2$ overnight. The antibody was diluted with a DMEM medium (5% human plasma), and 100 μL of serially diluted experimental drug (100 nM, 50 nM, 10 nM, 5 nM, 1 nM, 0.5 nM, 0.1 nM, 0.05 nM, and 0.01 nM based on the working concentration of the antibody) was added. A blank control group was designed, with three replicate wells set for each group. The plate was incubated in an incubator at 37 °C with 5% $CO_2$ for 6 days. After the incubation was completed, the 96-well plate was taken out and centrifuged at 350× g for 5 min, and then 135 μL of the supernatant was discarded by pipetting. 65 μL of CellTiter-Glo (Promega, Cat. No. G8461) was added. The plate was incubated at room temperature for 20 min. Then the fluorescence value was detected by a multi-label microplate tester.

3. Experimental results

The results are shown in FIG. 3.

The results showed that under the same experimental conditions, IgG-KA0012 had weaker non-specific killing as compared to the isotype control (IgG-KA0010, produced by Akeso Biopharma Inc.). This indicates that Mc-AAA-Dxd is more stable than Mc-GGFG-Dxd.

Example 4: Assay of Plasma Stability of Anti-HER3 Antibody-Drue Conjugate KA0013

1. Experimental drug

KA0013,
KA0011.

2. Experimental method

After the human venous blood was collected, the heparin sodium anticoagulant tube was gently inverted for uniform mixing, and centrifuged at 3500 rpm for 10 min at room temperature. The supernatant was plasma. The experimental drugs (at a working concentration: 100 μg/mL) were diluted with human plasma, and the diluted samples were incubated in an incubator at 37 °C with 5% $CO_2$ for 0 h, 24 h, 48 h, 72 h, 96 h, and 139 h. After the incubation was completed, 150 μL of the samples were taken out and quickly frozen in liquid nitrogen. After all the samples were collected, changes in free Dxd in plasma were detected by mass spectrometry.

Sample preparation for mass spectrometry: An appropriate amount of the above sample was taken and mixed with acetonitrile in a ratio of 1:1 to precipitate the protein. The mixture was centrifuged at 12,000 rpm for 3 min, and 10 μL of the supernatant was taken for mass spectrometry detection. Chromatographic conditions: column: MabPac RP (2.1 × 50 mm, 4 μm); mobile phase A: 0.1% formic acid in water; mobile phase B: 0.1% formic acid in acetonitrile; and column temperature: 30 °C.

The gradient is shown in Table 3 below.

Table 3

| Time (min) | Flow rate (mL/min) | A (%) | B (%) |
|---|---|---|---|
| 0 | 0.4 | 95 | 5 |
| 1 | 0.4 | 95 | 5 |
| 5 | 0.4 | 10 | 90 |
| 6 | 0.4 | 10 | 90 |
| 6.5 | 0.4 | 95 | 5 |

(continued)

| Time (min) | Flow rate (mL/min) | A (%) | B (%) |
|---|---|---|---|
| 7 | 0.4 | 10 | 90 |
| 8 | 0.4 | 10 | 90 |
| 8.1 | 0.4 | 95 | 5 |
| 10 | 0.4 | 95 | 5 |

3. Experimental results

The results are shown in FIG. 4.

The results showed that under the same experimental conditions, the amount of free Dxd in plasma in the KA0013 group was lower than that in the KA0011 group. This indicates that the stability of KA0013 in plasma is superior to that of KA0011.

Example *5: In Vitro* Assay of Cleavage Efficiency of Different Linker-Payloads by Cathepsin B

1. Experimental sample:

KA0010,
KA0012.

2. Experimental method

PBS (20× solution) (Sangon, Cat. No. B548117) was subjected to 20-fold dilution to obtain a PBS working solution. Cathepsin B (CTSB) (Sinobio, Cat. No. 10483-H08H) and linker-payload were diluted with the PBS working solution. 150 μL of CTSB (at a working concentration of 3 nM) was mixed uniformly with an equal volume of linker-payload (at a working concentration of 10 μM), and the mixture was incubated in an incubator at 37 °C with 5% $CO_2$ for 0 h, 0.5 h, 1 h, 2 h, and 4 h. After the incubation was completed, Halt™ Protease Inhibitor Cocktail, EDTA-Free (30X) (Thermofisher, Cat. No. 87785) was added to terminate the reaction.

The digested samples were directly loaded for mass spectrometry detection. Chromatographic conditions: column: MabPac RP (2.1 × 50 mm, 4 μm); mobile phase A: 0.1% formic acid in water; mobile phase B: 0.1% formic acid in acetonitrile; loading amount: 1 μL of 10 μM sample; and column temperature: 30 °C.

The gradient is shown in Table 4 below.

Table 4

| Time (min) | Flow rate (mL/min) | A (%) | B (%) |
|---|---|---|---|
| 0 | 0.4 | 95 | 5 |
| 1 | 0.4 | 95 | 5 |
| 5 | 0.4 | 10 | 90 |
| 6 | 0.4 | 10 | 90 |
| 6.5 | 0.4 | 95 | 5 |
| 7 | 0.4 | 10 | 90 |
| 8 | 0.4 | 10 | 90 |
| 8.1 | 0.4 | 95 | 5 |
| 10 | 0.4 | 95 | 5 |

3. Experimental results

The results are shown in FIG. 5.

The results showed that under the same experimental conditions, KA0012 (Mc-AAA-Dxd) and KA0010 (Mc-GGFG-Dxd) could both be efficiently cleaved by CSTB and effectively release the payload, with identical efficiency.

Example 6: Assay for Binding Activity of Anti-HER3 Antibody-Drug Conjugate KA0013 to Antigen by ELISA

The Elisa plates were coated with human HER3-his and incubated, and the test antibodies were added after blocking. After incubation and washing, goat anti-human IgG Fc, HRP (purchased from Jackson ImmunoResearch Inc., Cat. No. 109-035-098) was added. After incubation and washing, TMB (Neogen, 308177) was added for chromogenic reaction. After the reaction was terminated, the absorbance at 450 nm was detected in a microplate reader. The data were analyzed and processed using SoftMax Pro 6.2.1 software.
The results are shown in FIG. 6 and Table 5.

Table 5: Binding activity of KA0013 and KA0011 for antigen human HER3-his

| Coating: huHER3-his(0.5μg/mL, 50μL/well) | | | | | | |
|---|---|---|---|---|---|---|
| Antibody dilution (nM) | KA0013 | | KA0011 | | Patritumab | |
| 1.000 | 2.848 | 2.861 | 2.804 | 2.819 | 2.869 | 2.844 |
| 0.333 | 2.883 | 2.900 | 2.814 | 2.829 | 2.891 | 2.856 |
| 0.111 | 2.820 | 2.841 | 2.756 | 2.748 | 2.831 | 2.795 |
| 0.037 | 2.538 | 2.587 | 2.390 | 2.438 | 2.569 | 2.570 |
| 0.012 | 1.702 | 1.755 | 1.508 | 1.531 | 1.780 | 1.775 |
| 0.004 | 0.819 | 0.847 | 0.718 | 0.700 | 0.870 | 0.872 |
| 0.001 | 0.362 | 0.370 | 0.306 | 0.315 | 0.382 | 0.394 |
| 0 | 0.082 | 0.092 | 0.075 | 0.078 | 0.091 | 0.087 |
| Secondary antibody: Goat Anti Human IgG Fc, HRP(1: 5000) | | | | | | |
| $EC_{50}$ (nM) | 0.009 | | 0.011 | | 0.009 | |

The results showed that the anti-HER3 antibody-drug conjugates KA0013 and KA0011, and patritumab could all effectively bind to the antigen human HER3-his in a dose-dependent manner.

Example 7: Determination of Kinetic Parameters of Anti-HER3 Antibody-Drug Conjugates

1. Experimental sample

KA0013
KA0011

2. Experimental method

The dilution buffer for experimental samples was PBS containing 0.02% Tween-20 and 0.1% BSA (pH 7.4). 1 μg/mL huHER3-his-Biotin was immobilized on the SA sensor at an immobilization height of 1 nm. The sensor was equilibrated in a buffer for 60 s, and then the binding of the immobilized huHER3-his-Biotin on the sensor to the antibodies at concentrations of 0.2469-20 nM (three-fold dilution) was determined for 120 s. The antibodies were then dissociated in the buffer for 300 s. The sample plate shaking speed was 1000 rpm, the determination temperature was 30 °C, and the frequency was 5.0 Hz. The data were analyzed by 1:1 model fitting to obtain affinity constants. The data acquisition software was Fortebio Data Acquisition 12.0, and the data analysis software was Fortebio Data Analysis 12.0.

3. Experimental results

The results are shown in Table 6 and FIGs. 7 to 8.

Table 6: Kinetic parameters for binding of anti-HER3 antibody-drug conjugates to human HER3-his-biotin

| Sample | KD (M) | kon (1/Ms) | S E (kon) | kdis (1/s) | S E (kdis) | Rmax (nm) |
|---|---|---|---|---|---|---|
| KA0013 | 8.08E-11 | 1.49E+06 | 4.63E+03 | 1.21E-04 | 2.77E-06 | 0.116-0.243 |
| KA0011 | 5.52E-11 | 1.40E+06 | 6.33E+03 | 7.70E-05 | 3.99E-06 | 0.128-0.197 |

KD is affinity constant; kon is binding rate of antigen and antibody; kdis is dissociation rate of antigen and antibody; KD = kdis/kon.

The results showed that the anti-HER3 antibody-drug conjugates KA0013 and KA0011 both had good affinity for the antigen human HER3-his-biotin.

Example 8: Assay for Specific Cell Killing Activity of Anti-HER3 Antibody-Drug Conjugates

1. Experimental drug

KA0013
KA0011
IgG-KA0012

2. Experimental method

NCI-H1781 cells (Cobioer Biosciences Co., Ltd., Cat. No. CBP60116) were collected, and centrifuged at 1000× g for 5 min, and then the supernatant was removed. The cells were resuspended and counted, and the viability was determined. The cells were diluted, and NCI-H1781 (2500 cells/100 μL) was added to each well of a black-bottom 96-well plate. The plate was incubated in an incubator at 37 °C with 5% $CO_2$ overnight. The experimental drug was diluted with a medium (10% human ab serum), and 100 μL of the experimental drug was added to each well according to the experimental design. A blank control was designed, with three replicate wells set for each group. The plate was incubated in an incubator at 37 °C with 5% $CO_2$ for 6 days. After incubation, the 96-well plate was centrifuged at 350× g for 5 min, and then 135 μL of the supernatant was discarded. 60 μL of CellTiter-Glo (Promega, Cat. No. G8461) was added to each well. The plate was incubated at room temperature for 15 min. Then the fluorescence value was detected by a multi-label microplate tester.

3. Experimental results

The results are shown in FIG. 9. The results showed that the anti-HER3 antibody-drug conjugates KA0013 and KA0011 could all specifically kill NCI-H1781 cells.

Example 9: Study on ADCC Activity of Anti-HER3 Antibody-Drug Conjugates

MDA-MB-453 cells (purchased from Cobioer Biosciences Co., Ltd., Cat. No. CBP60386) and PBMCs (provided by Zhongshan Blood Center) in the logarithmic growth phase were collected and centrifuged at 170× g for 5 min, and the supernatant was discarded. The cell pellet was resuspended in an assay medium (RPMI-1640 containing 1% FBS), centrifuged and washed twice, and the cells were counted. The cell density was adjusted with the assay medium, and the target cell suspension was seeded into a 96-well plate (containing about $3 \times 10^4$ target cells/well) at 50 μL/well. Then, serially diluted drugs (5 μg/mL, 15 μg/mL, and 45 μg/mL based on the working concentration of the antibody, respectively) were added, and the plate was pre-incubated at room temperature for 1 h. After the pre-incubation was completed, the PBMC suspension was added to the sample wells at 100 μL/well (each well contained about $9 \times 10^5$ PBMCs with an effector-to-target ratio of 30:1), mixed uniformly, and incubated in an incubator at 37 °C with 5% $CO_2$ for 4 h. After the incubation was completed, the 96-well plate was taken out and centrifuged at 250× g for 5 min. 100 μL of culture supernatant was carefully pipetted and transferred to another 96-well plate. The freshly prepared reaction solution was added at 100 μL/well according to the instructions of the Cytotoxicity Detection Kit (Roche, Cat. No. 11644793001), and the plate was incubated at room temperature for 30 min in the dark. OD values at 490 nm and 650 nm were measured. The OD value of each group = $OD_{490\,nm}$ - $OD_{650\,nm}$. The ADCC activity (expressed as ADCC%) was calculated based on the measured OD value of each group.

ADCC% = (OD of experimental group - average OD of target cell spontaneous group)/(average OD of positive control - average OD of target cell spontaneous group) × 100%

The results are shown in FIG. 10. The results showed that in the MDA-MB-453 target cell system, KA0011 had no ADCC activity for MDA-MB-453 cells, while KA0013 had ADCC activity for MDA-MB-453 cells.

Example 10: *In Vivo* Efficacy Assay of Anti-HER3 Antibody-Drug Conjugates

To determine the anti-tumor activity of the anti-HER3 antibody-drug conjugates *in vivo,* HCC827 human lung cancer cells (purchased from GuangZhou Jennio Biotech Co., Ltd.) were first inoculated subcutaneously into 5- to 8-week-old female CB-17 SCID mice (purchased from Guangdong Model Organisms Center, Inc.) on the upper posterior region of the right hind thigh. The day of grouping was defined as D0. The route of administration was intravenous injection (iv), once a week for a total of 3 doses. The modeling and specific dosing regimen are shown in Table 7. After the administration, the length and width of the tumors in each group were measured, and the tumor volume was calculated.

Table 7: Dosing regimen of anti-HER3 antibody-drug conjugates for treatment of CB-17 SCID mouse model of HCC827 human lung cancer cell xenograft tumor

| Group | Number of animals | Modeling | Dosing regimen |
|---|---|---|---|
| hIgG1 (1.5 mg/kg) group | 6 | HCC827 cells were inoculated subcutaneously at 6 × $10^6$ cells/200 μL/mouse into CB-17 SCID mice on the upper posterior region of the right hind thigh. | hIgG1 (1.5mg/kg), IV, weekly×3 |
| IgG-KA0012 (1.5 mg/kg) group | 6 | | IgG-KA0012, 1.5mg/kg, IV, weekly×3 |
| Patritumab (3 mg/kg) group | 6 | | Patritumab, 3mg/kg, IV, weekly×3 |
| KA0013 (3 mg/kg) group | 6 | | KA0013, 3mg/kg, IV, weekly×3 |
| KA0013 (1.5 mg/kg) group | 6 | | KA0013, 1.5mg/kg, IV, weekly×3 |

The results are shown in FIG. 11. The results showed that the anti-HER3 antibody-drug conjugate KA0013 could effectively inhibit the growth of mouse tumors compared to the isotype control antibody. In addition, as shown in FIG. 12, the tumor-bearing mice exhibited good tolerance to test drug KA0013, and the body weights of the tumor-bearing mice in each group were not affected.

Although the specific embodiments of the present disclosure have been described in detail, those skilled in the art will understand that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. A compound or a pharmaceutically acceptable salt thereof, wherein the compound comprises a compound of Formula III, n alanine residues, and a compound of Formula IV that are sequentially linked,

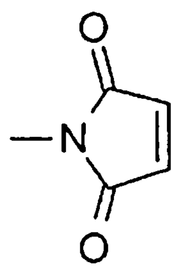

Formula III,

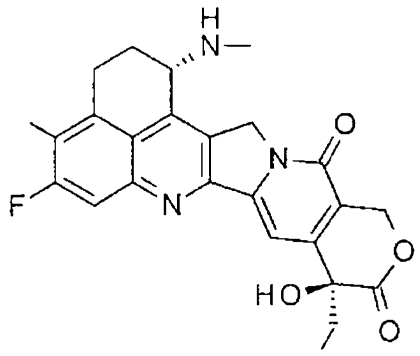

Formula IV,

wherein

n is 2, 3, 4, 5, or 6;
any two of the compound of Formula III, the n alanine residues, and the compound of Formula IV are independently either linked directly or via a chemical group.

2. The compound or the pharmaceutically acceptable salt thereof according to claim 1, having a structural formula represented by Formula I below,

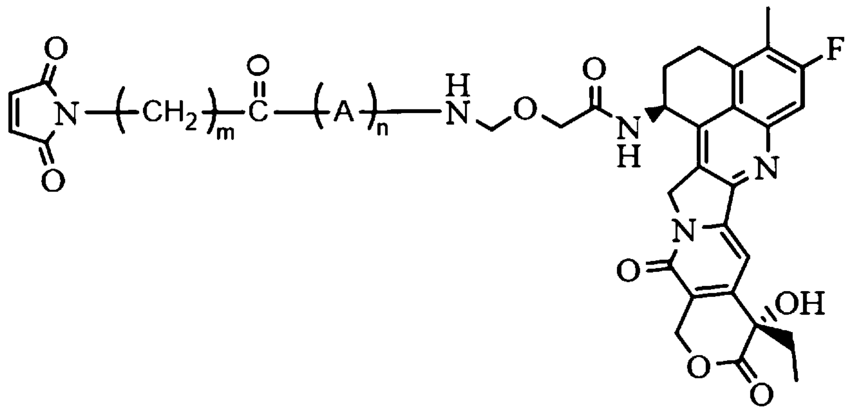

Formula I

wherein

A represents alanine;
m is 2, 3, 4, 5, 6, 7, 8, 9, 10, 11, or 12;
n is 2, 3, 4, 5, or 6.

3. The compound or the pharmaceutically acceptable salt thereof according to any one of claims 1 to 2, wherein the compound is represented by Formula II below,

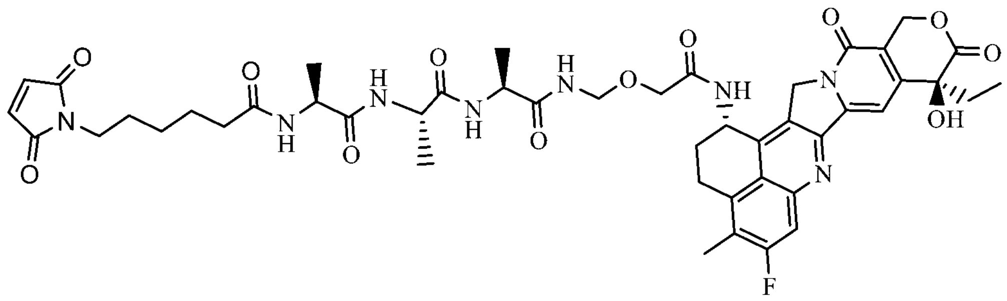

Formula II

.

4. An antibody-drug conjugate, comprising an antibody or an antigen-binding fragment thereof, a linker, and a payload, wherein

the linker and the payload are the compound or the pharmaceutically acceptable salt thereof according to any one of claims 1 to 3;
preferably, the linker is linked to the antibody or the antigen-binding fragment thereof via one or more thioether bonds;
preferably, the linker forms a thioether bond with a sulfur atom at a disulfide bond position of the hinge portion of the antibody.

5. The antibody-drug conjugate according to claim 4, wherein the antibody is an IgG antibody, preferably an IgG1 antibody.

6. The antibody-drug conjugate according to any one of claims 4 to 5, wherein the antibody is an anti-HER3 antibody.

7. The antibody-drug conjugate according to any one of claims 4 to 6, wherein the antibody comprises a heavy chain variable region and a light chain variable region, wherein

the heavy chain variable region comprises an HCDR1 set forth in SEQ ID NO: 1, an HCDR2 set forth in SEQ ID NO: 2, and an HCDR3 set forth in SEQ ID NO: 3;
the light chain variable region comprises an LCDR1 set forth in SEQ ID NO: 4, an LCDR2 set forth in SEQ ID NO: 5, and an LCDR3 set forth in SEQ ID NO: 6.

8. The antibody-drug conjugate according to claim 7, wherein the heavy chain variable region comprises an amino acid sequence set forth in SEQ ID NO: 7, and the light chain variable region comprises an amino acid sequence set forth in SEQ ID NO: 9.

9. The antibody-drug conjugate according to any one of claims 4 to 8, wherein a heavy chain constant region of the antibody is an Ig gamma-1 chain C region or an Ig gamma-4 chain C region, and a light chain constant region is an Ig kappa chain C region.

10. The antibody-drug conjugate according to any one of claims 4 to 9, wherein the heavy chain constant region of the antibody comprises an amino acid sequence set forth in SEQ ID NO: 11, and the light chain constant region comprises an amino acid sequence set forth in SEQ ID NO: 13.

11. The antibody-drug conjugate according to any one of claims 4 to 10, wherein an average number of linker-payloads conjugated to each antibody molecule is 1-8.

12. The antibody-drug conjugate according to any one of claims 4 to 11, having ADCC activity.

13. The antibody-drug conjugate according to any one of claims 4 to 12, wherein
an $EC_{50}$ for binding of the antibody-drug conjugate to an antigen targeted by the antibody-drug conjugate is identical to

or substantially identical to an $EC_{50}$ for binding of an antibody or an antigen-binding fragment thereof comprised in the antibody-drug conjugate to the same antigen.

**14.** A pharmaceutical composition, comprising the antibody-drug conjugate according to any one of claims 4 to 13 and one or more pharmaceutically acceptable auxiliary materials.

**15.** Use of the antibody-drug conjugate according to any one of claims 4 to 13 in the preparation of a medicament for treating or preventing a tumor, wherein preferably, the tumor is an HER3-positive tumor;

preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon cancer, rectal cancer, uterine cancer, salivary gland cancer, vulvar cancer, thyroid cancer, anal cancer, and penile cancer;
preferably, the lung cancer is non-small cell lung cancer;
preferably, the colon cancer is metastatic colon cancer.

**16.** The antibody-drug conjugate according to any one of claims 4 to 13 for use in treating or preventing a tumor, wherein

preferably, the tumor is an HER3-positive tumor;
preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon cancer, rectal cancer, uterine cancer, salivary gland cancer, vulvar cancer, thyroid cancer, anal cancer, and penile cancer;
preferably, the lung cancer is non-small cell lung cancer;
preferably, the colon cancer is metastatic colon cancer.

**17.** A method for treating or preventing a tumor, comprising a step of administering to a subject in need an effective amount of the antibody-drug conjugate according to any one of claims 4 to 13, wherein

preferably, the tumor is an HER3-positive tumor;
preferably, the tumor is selected from one or more of lung cancer, clear cell sarcoma, skin cancer, renal cancer, urothelial cancer, prostate cancer, glioblastoma multiforme, ovarian cancer, pancreatic cancer, breast cancer, melanoma, liver cancer, bladder cancer, gastric cancer, gastrointestinal stromal tumor, cervical cancer, head and neck cancer, esophageal cancer, epidermoid carcinoma, peritoneal cancer, adult glioblastoma multiforme, colon cancer, rectal cancer, uterine cancer, salivary gland cancer, vulvar cancer, thyroid cancer, anal cancer, and penile cancer;
preferably, the lung cancer is non-small cell lung cancer;
preferably, the colon cancer is metastatic colon cancer.

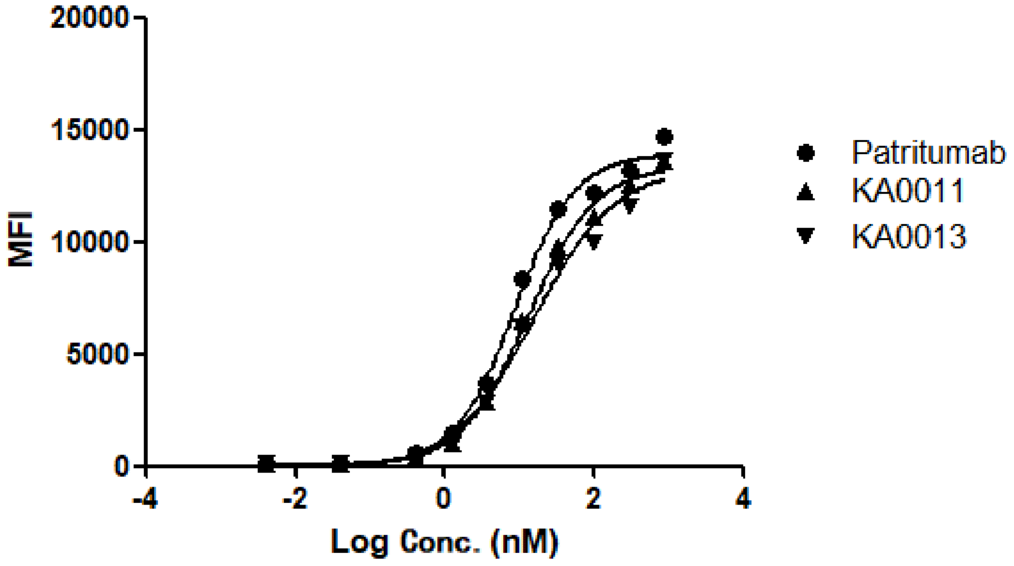
20000
15000
10000
5000
0
MFI
-4
-2
0
2
4
Log Conc. (nM)
Patritumab
KA0011
KA0013

FIG. 1

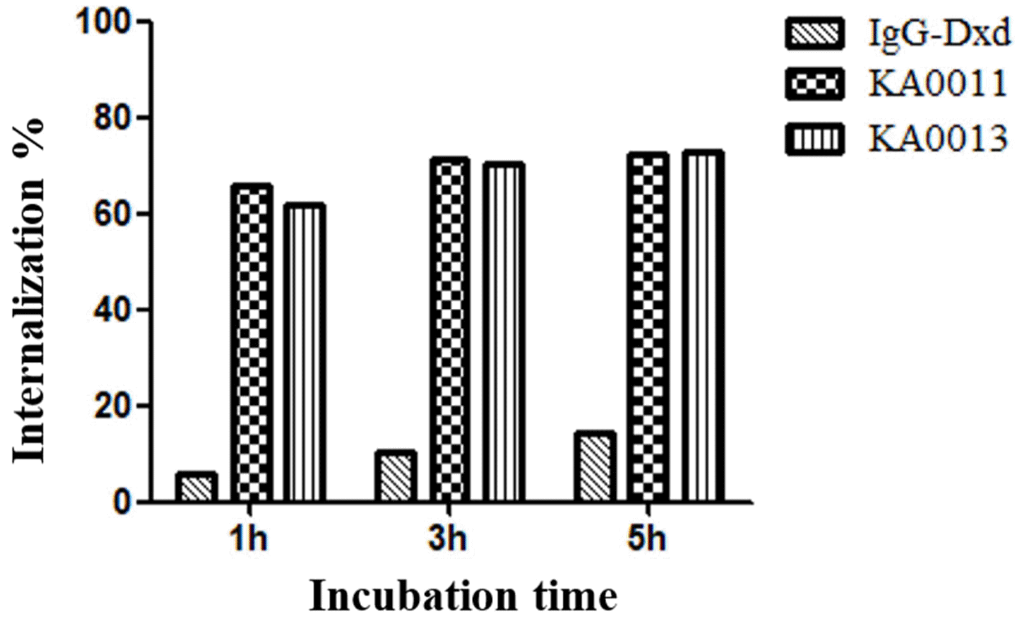
100
80
60
40
20
0
Internalization %
1h
3h
5h
Incubation time
IgG-Dxd
KA0011
KA0013

FIG. 2

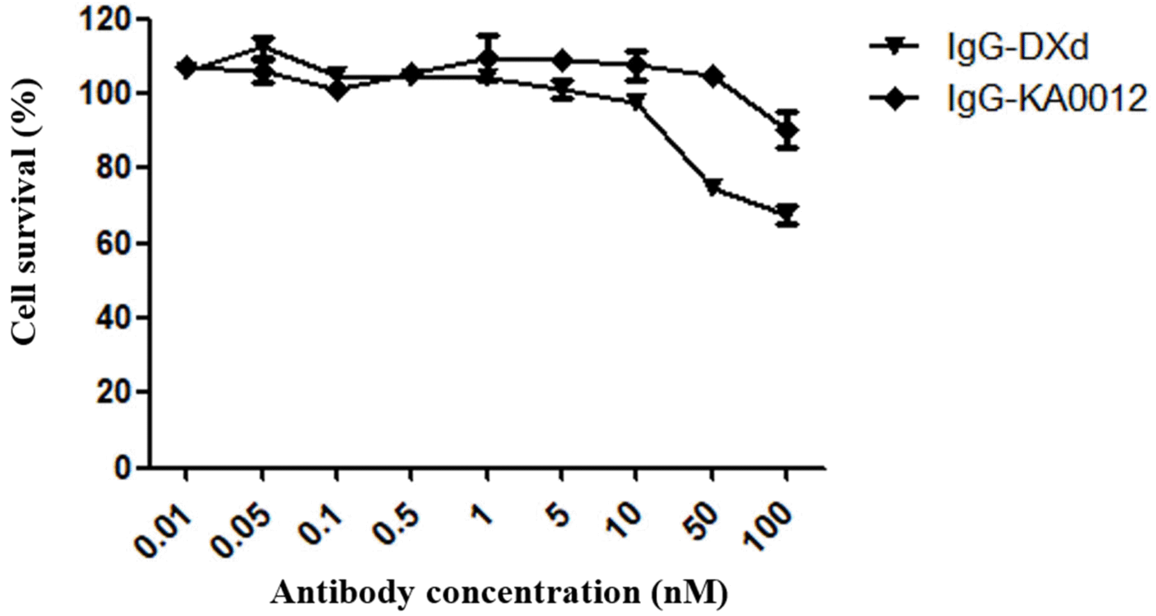
Cell survival (%)
120
100
80
60
40
20
0
0.01
0.05
0.1
0.5
1
5
10
50
100
Antibody concentration (nM)
IgG-DXd
IgG-KA0012

FIG. 3

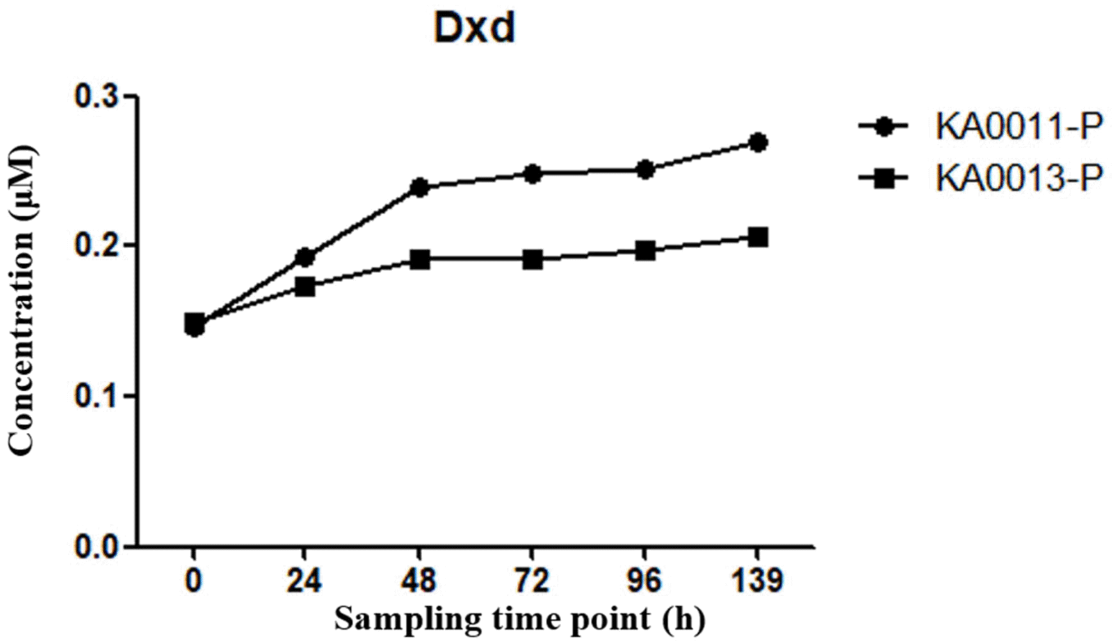
Dxd
Concentration (μM)
0.3
0.2
0.1
0.0
0
24
48
72
96
139
Sampling time point (h)
KA0011-P
KA0013-P

FIG. 4

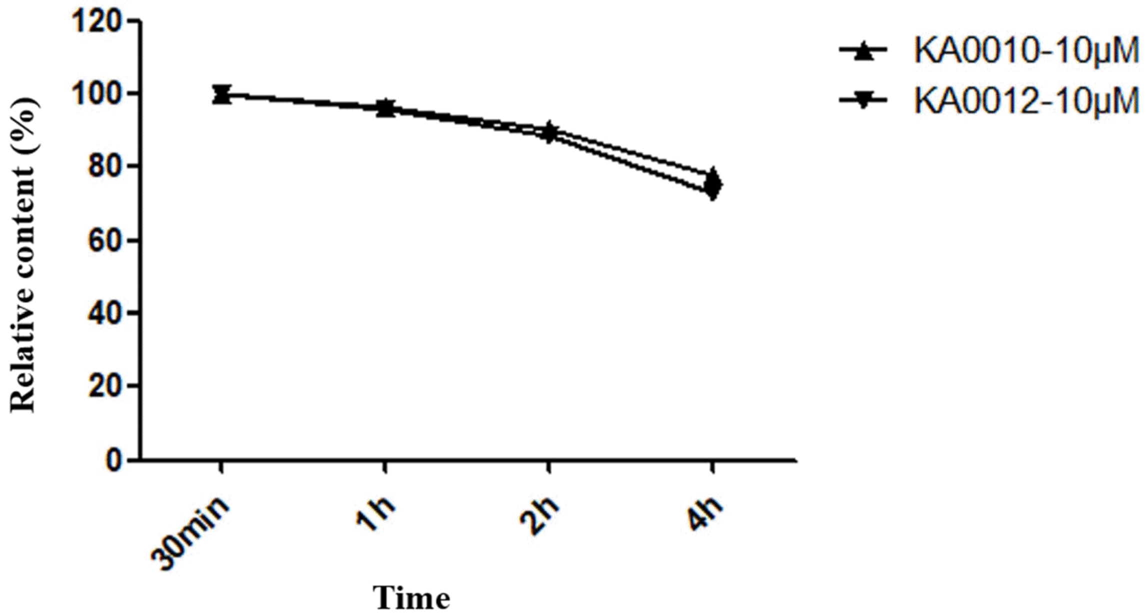
120
100
80
60
40
20
0
Relative content (%)
30min
1h
2h
4h
Time
KA0010-10μM
KA0012-10μM

FIG. 5

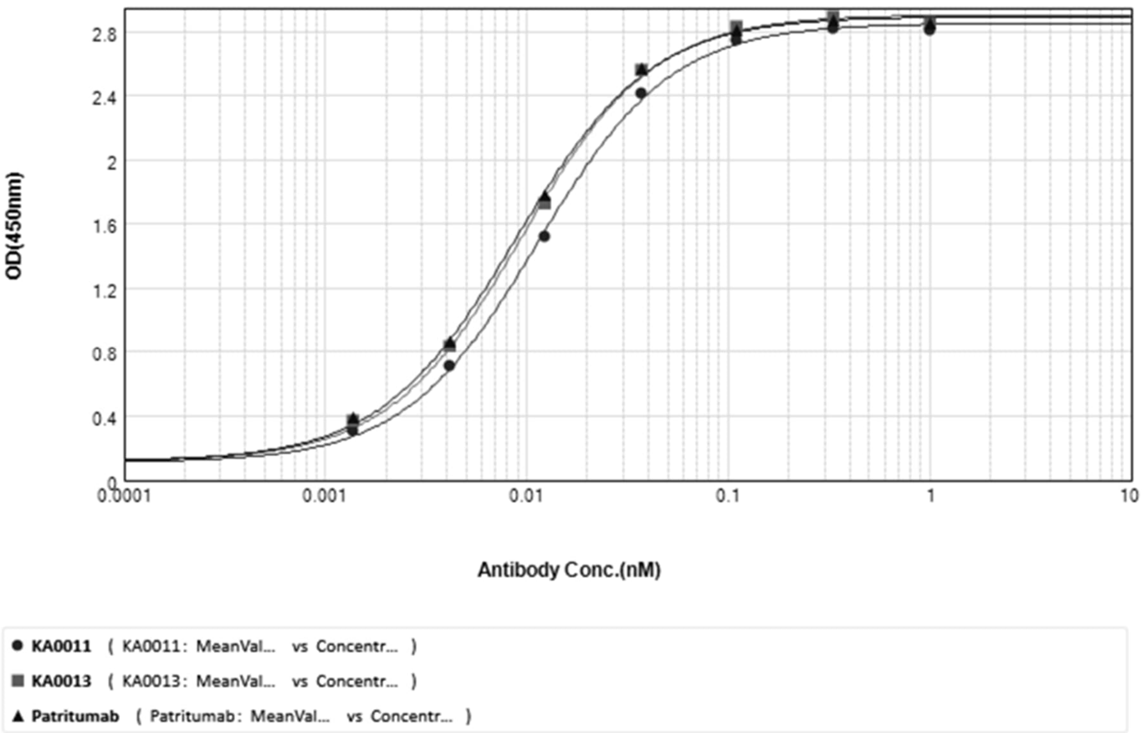


Curve Fit Results ▲

Curve Fit : 4-Parameter $y = D + \frac{A - D}{1 + \left(\frac{x}{C}\right)^B}$

| | Parameter | Estimated Value | Std. Error | Confidence Interval |
|---|---|---|---|---|
| **KA0011** $R^2 = 0.999$ EC50 = 0.011 | A | 0.111 | 0.040 | [4.29e-4, 0.221] |
| | B | 1.310 | 0.079 | [1.091, 1.528] |
| | C | 0.011 | 5.94e-4 | [0.010, 0.013] |
| | D | 2.855 | 0.032 | [2.765, 2.944] |
| **KA0013** $R^2 = 0.999$ EC50 = 0.009 | A | 0.119 | 0.043 | [4.15e-5, 0.238] |
| | B | 1.319 | 0.081 | [1.095, 1.543] |
| | C | 0.009 | 5.06e-4 | [0.008, 0.011] |
| | D | 2.909 | 0.032 | [2.819, 2.998] |
| **Patritumab** $R^2 = 0.999$ EC50 = 0.009 | A | 0.118 | 0.038 | [0.013, 0.223] |
| | B | 1.300 | 0.069 | [1.108, 1.492] |
| | C | 0.009 | 4.23e-4 | [0.008, 0.010] |
| | D | 2.897 | 0.028 | [2.819, 2.975] |

FIG. 6

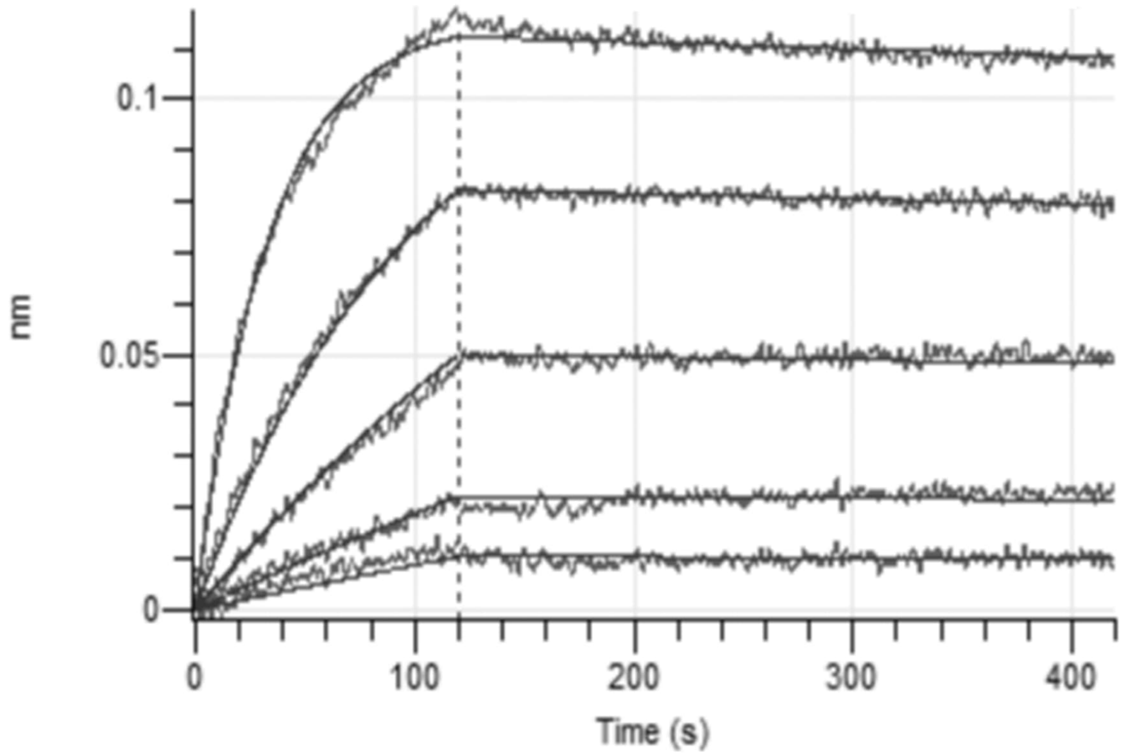
0.1
0.05
0
nm
0
100
200
300
400
Time (s)

FIG. 7

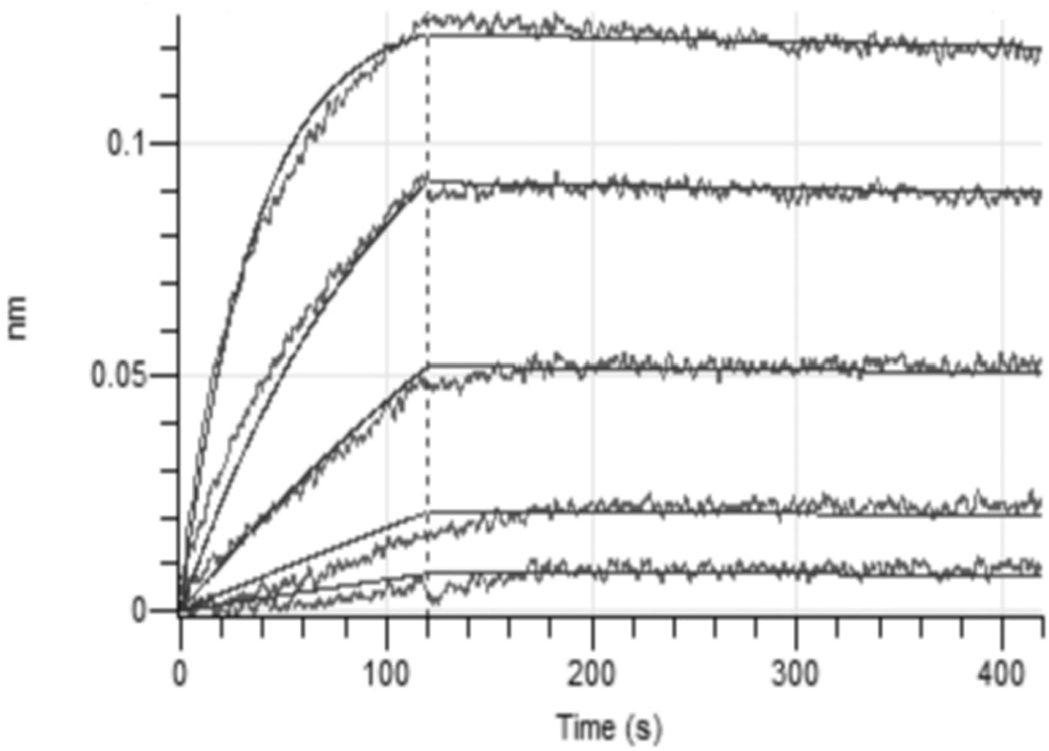
0.1
0.05
0
nm
0
100
200
300
400
Time (s)

FIG. 8

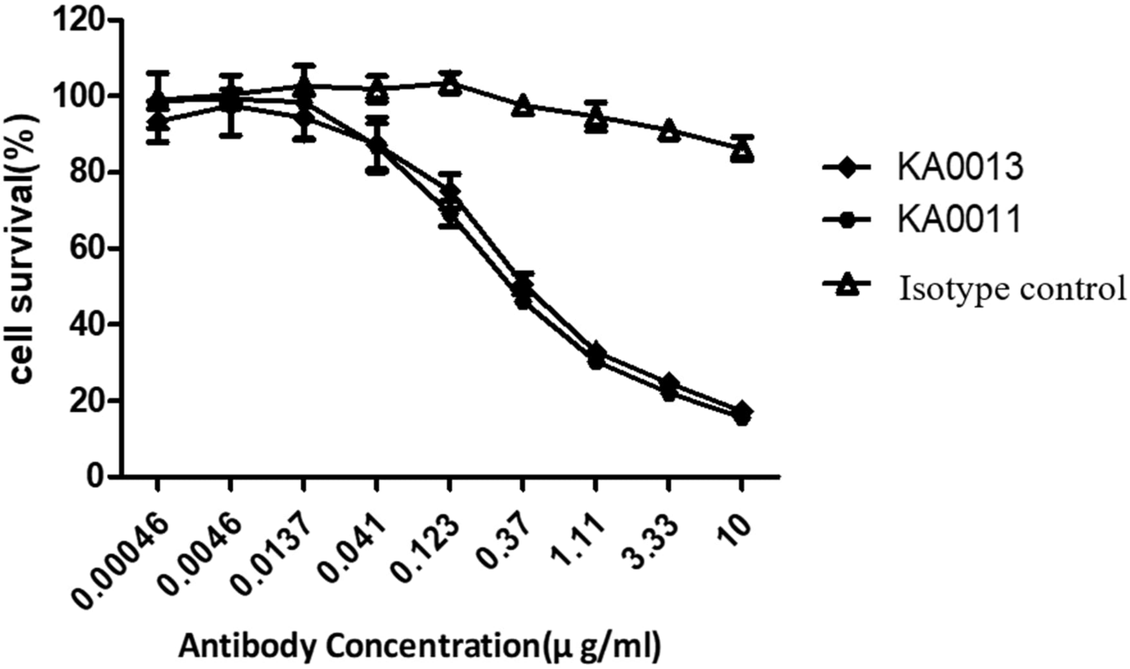
120
100
80
60
40
20
0
cell survival(%)
KA0013
KA0011
Isotype control
0.00046
0.0046
0.0137
0.041
0.123
0.37
1.11
3.33
10
Antibody Concentration(μ g/ml)

FIG. 9

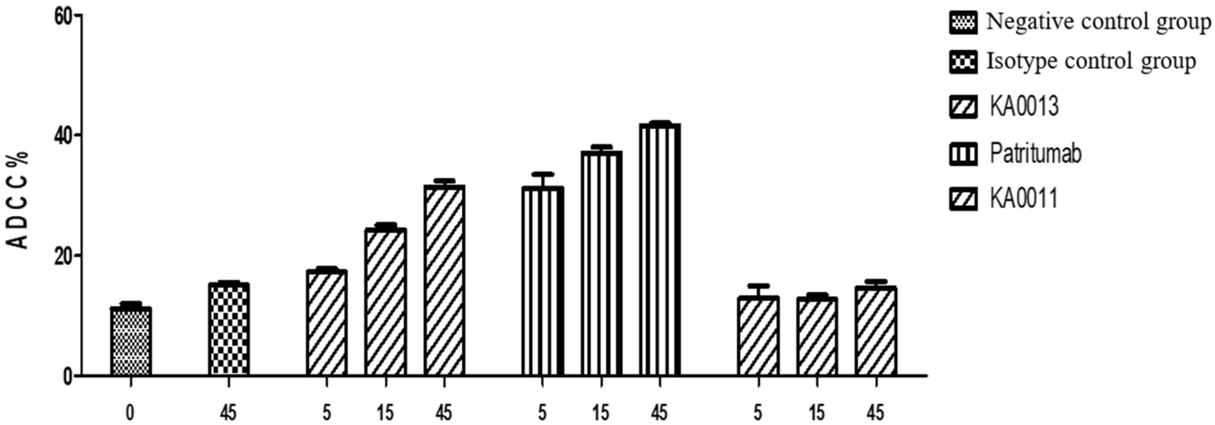
60
40
20
0
ADCC%
Negative control group
Isotype control group
KA0013
Patritumab
KA0011
0
45
5
15
45
5
15
45
5
15
45
Working concentration (μg/mL)

FIG. 10

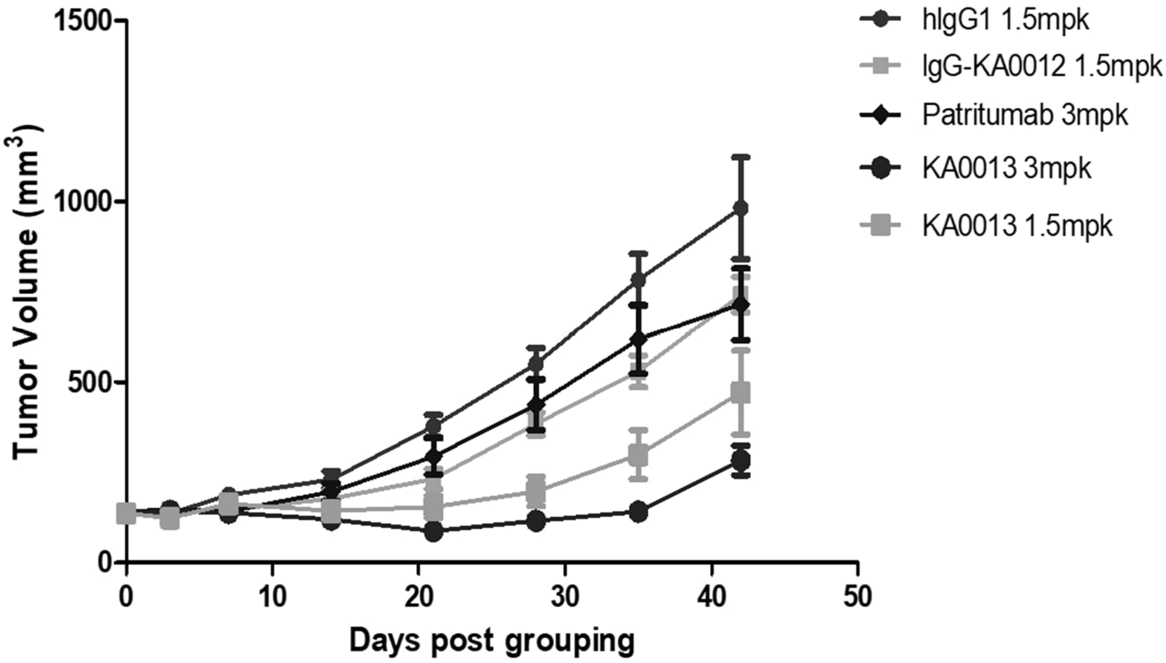
1500
1000
500
0
Tumor Volume (mm$^3$)
0
10
20
30
40
50
Days post grouping
hIgG1 1.5mpk
IgG-KA0012 1.5mpk
Patritumab 3mpk
KA0013 3mpk
KA0013 1.5mpk

FIG. 11

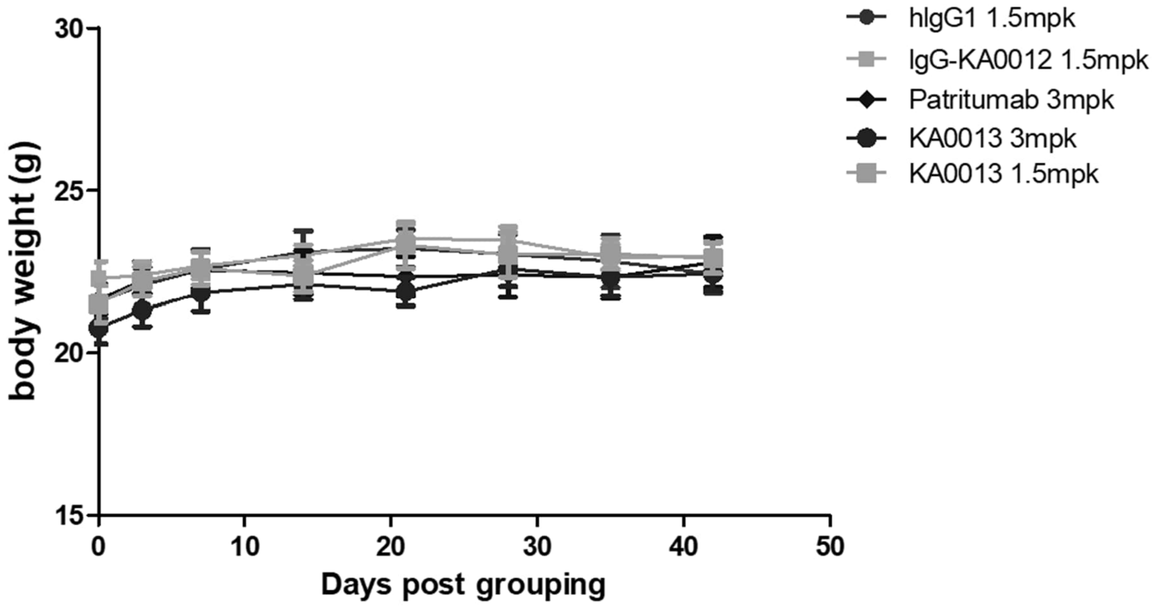
30
25
20
15
body weight (g)
0
10
20
30
40
50
Days post grouping
hIgG1 1.5mpk
IgG-KA0012 1.5mpk
Patritumab 3mpk
KA0013 3mpk
KA0013 1.5mpk

FIG. 12

**INTERNATIONAL SEARCH REPORT**

International application No.
**PCT/CN2024/112677**

**A. CLASSIFICATION OF SUBJECT MATTER**

A61K39/395(2006.01)i; C07K5/062(2006.01)i; A61K31/4745(2006.01)i; A61K31/48(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, WOTXT, JPTXT, CNKI, ISI Web of Science, 万方数据检索系统, Wanfang Data Search System, pubmed, patentics, 中国专利生物序列检索系统, China Patent Biological Sequence Search System, GENBANK, STN: SEQ ID NOs: 1-16, ADC, antibody drug conjugate, 抗体偶联药, 抗体药物复合物, 单抗偶联物, 抗体化疗药物偶联物, U3-1402, HER3-Dxd, patritumab deruxtecan, patritumab-GGFG-Dxd, Mc-GGFG-Dxd, 马来酰亚胺, 马来酰亚胺-N-基, 马来酰亚胺己酰基, maleimide, 顺丁烯二酰亚胺, 顺丁烯酰亚胺, maleimide caproyl, HER3抗体, patritumab, Dxd, deruxtecan, 德鲁替康, 1599440-33-1, 依喜替康, DX-8951, 丙氨酸, Ala, Alanine, 中山康方生物医药有限公司, 李百勇, 夏瑜, 王忠民, 张鹏

**C. DOCUMENTS CONSIDERED TO BE RELEVANT**

| Category* | Citation of document, with indication, where appropriate, of the relevant passages | Relevant to claim No. |
|---|---|---|
| X | WO 2023131219 A1 (VIRTUOSO BINCO, INC.) 13 July 2023 (2023-07-13) abstract, claims 1, 48, 51, 82, 108-113 and 124-137, and description, paragraphs 144-145, and table 7 | 1-5, 9, 11-17 |
| Y | WO 2023131219 A1 (VIRTUOSO BINCO, INC.) 13 July 2023 (2023-07-13) abstract, claims 1, 48, 51, 82, 108-113 and 124-137, and description, paragraphs 144-145, and table 7 | 6-17 |
| Y | CN 111228511 A (DAICHI SANKYO COMPANY, LTD. et al.) 05 June 2020 (2020-06-05) claims 1-12, and description, paragraph 482, and sequence listing | 6-17 |
| A | CN 104755494 A (DAIICHI SANKYO COMPANY, LIMITED) 01 July 2015 (2015-07-01) claims 1-49 | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 November 2024** | **21 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/112677**

**C. DOCUMENTS CONSIDERED TO BE RELEVANT**

| Category* | Citation of document, with indication, where appropriate, of the relevant passages | Relevant to claim No. |
|---|---|---|
| A | CN 106163559 A (DAICHI SANKYO COMPANY, LTD. et al.) 23 November 2016 (2016-11-23) claims 1-38 | 1-17 |
| A | CN 110464847 A (DAIICHI SANKYO COMPANY, LIMITED) 19 November 2019 (2019-11-19) claims 1-27 | 1-17 |
| A | US 2017035906 A1 (DAIICHI SANKYO COMPANY, LIMITED) 09 February 2017 (2017-02-09) claims 1-32 | 1-17 |
| A | WO 2020240467 A1 (DAIICHI SANKYO COMPANY, LIMITED) 03 December 2020 (2020-12-03) claims 1-66 | 1-17 |

Form PCT/ISA/210 (second sheet) (July 2022)

**INTERNATIONAL SEARCH REPORT**

International application No.
**PCT/CN2024/112677**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT

International application No.
PCT/CN2024/112677

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

Claim 17 relates to a method for treating or preventing cancer, which falls within subject matter for which no search is required by the International Searching Authority as defined in PCT Rule 39.1(iv): (4) methods for treatment of the human or animal body by surgery or therapy, as well as diagnostic methods. The international search is carried out on the basis of the use in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| WO 2023131219 A1 | 13 July 2023 | None | |
| CN 111228511 A | 05 June 2020 | None | |
| CN 104755494 A | 01 July 2015 | US 2016279259 A1 | 29 September 2016 |
| | | US 9808537 B2 | 07 November 2017 |
| | | LT 2907824 T | 11 June 2018 |
| | | BR 122021014396 B1 | 05 July 2022 |
| | | JP 2023011799 A | 24 January 2023 |
| | | JP 7523506 B2 | 26 July 2024 |
| | | JP 2022008581 A | 13 January 2022 |
| | | JP 7170812 B2 | 14 November 2022 |
| | | CA 3021435 A1 | 17 April 2014 |
| | | CA 3021435 C | 12 October 2021 |
| | | JP 6030267 B1 | 24 November 2016 |
| | | JP 2017036274 A | 16 February 2017 |
| | | BR 112015006521 A2 | 05 September 2017 |
| | | BR 112015006521 B1 | 03 March 2022 |
| | | SI 2907824 T1 | 29 June 2018 |
| | | NZ 746440 A | 29 November 2019 |
| | | KR 20220100727 A | 15 July 2022 |
| | | KR 102456726 B1 | 20 October 2022 |
| | | JP 5953378 B2 | 20 July 2016 |
| | | JPWO 2014057687 A1 | 05 September 2016 |
| | | PT 2907824 T | 07 June 2018 |
| | | PT 3342785 T | 25 February 2020 |
| | | CY 1122789 T1 | 05 May 2021 |
| | | US 2020282073 A1 | 10 September 2020 |
| | | US 11633493 B2 | 25 April 2023 |
| | | SG 10201804788 XA | 30 July 2018 |
| | | WO 2014057687 A1 | 17 April 2014 |
| | | CA 2885800 A1 | 17 April 2014 |
| | | CA 2885800 C | 04 December 2018 |
| | | ES 2773710 T3 | 14 July 2020 |
| | | US 2024082413 A1 | 14 March 2024 |
| | | US 2015297748 A1 | 22 October 2015 |
| | | US 10195288 B2 | 05 February 2019 |
| | | AU 2020200548 A1 | 13 February 2020 |
| | | AU 2020200548 B2 | 25 August 2022 |
| | | IL 302494 A | 01 June 2023 |
| | | IL 302494 B1 | 01 July 2024 |
| | | KR 20180030734 A | 23 March 2018 |
| | | KR 101901558 B1 | 21 September 2018 |
| | | MX 2020010964 A | 09 November 2020 |
| | | KR 20150067149 A | 17 June 2015 |
| | | KR 101841818 B1 | 26 March 2018 |
| | | TW 202233186 A | 01 September 2022 |
| | | TR 201809636 T4 | 23 July 2018 |
| | | HRP 20180870 T1 | 13 July 2018 |
| | | JP 2024147681 A | 16 October 2024 |
| | | CY 1120363 T1 | 10 July 2019 |
| | | SG 11201502887 WA | 28 May 2015 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | | | Publication date (day/month/year) |
|---|---|---|---|---|---|
| | | KR | 20180105271 | A | 27 September 2018 |
| | | KR | 102052319 | B1 | 05 December 2019 |
| | | KR | 20190135559 | A | 06 December 2019 |
| | | KR | 102087017 | B1 | 10 March 2020 |
| | | KR | 20240144454 | A | 02 October 2024 |
| | | EP | 3632471 | A1 | 08 April 2020 |
| | | MY | 170251 | A | 13 July 2019 |
| | | TW | 201811746 | A | 01 April 2018 |
| | | TWI | 650312 | B | 11 February 2019 |
| | | RS | 57278 | B1 | 31 August 2018 |
| | | DK | 3342785 | T3 | 23 March 2020 |
| | | PL | 3342785 | T3 | 07 September 2020 |
| | | TW | 201420117 | A | 01 June 2014 |
| | | TWI | 615152 | B | 21 February 2018 |
| | | TW | 202033499 | A | 16 September 2020 |
| | | TWI | 757740 | B | 11 March 2022 |
| | | KR | 20220029776 | A | 08 March 2022 |
| | | KR | 102417310 | B1 | 05 July 2022 |
| | | DK | 2907824 | T3 | 23 July 2018 |
| | | KR | 20210132240 | A | 03 November 2021 |
| | | KR | 102369419 | B1 | 02 March 2022 |
| | | PL | 2907824 | T3 | 31 August 2018 |
| | | PH | 12018501433 | A1 | 27 February 2019 |
| | | ZA | 201501825 | B | 25 May 2022 |
| | | HK | 1256631 | A1 | 27 September 2019 |
| | | KR | 20210038728 | A | 07 April 2021 |
| | | KR | 102320907 | B1 | 02 November 2021 |
| | | IL | 238143 | A0 | 31 May 2015 |
| | | IL | 238143 | B | 30 January 2020 |
| | | JP | 2016196484 | A | 24 November 2016 |
| | | JP | 6186045 | B2 | 23 August 2017 |
| | | KR | 20200026323 | A | 10 March 2020 |
| | | KR | 102237639 | B1 | 07 April 2021 |
| | | SI | 3342785 | T1 | 28 February 2020 |
| | | KR | 20230142808 | A | 11 October 2023 |
| | | KR | 102709509 | B1 | 24 September 2024 |
| | | NZ | 705394 | A | 26 October 2018 |
| | | RS | 60000 | B1 | 30 April 2020 |
| | | IL | 271760 | A | 27 February 2020 |
| | | IL | 271760 | B | 01 August 2022 |
| | | AU | 2013328111 | A1 | 12 March 2015 |
| | | AU | 2013328111 | B2 | 02 November 2017 |
| | | EP | 2907824 | A1 | 19 August 2015 |
| | | EP | 2907824 | A4 | 08 June 2016 |
| | | EP | 2907824 | B1 | 11 April 2018 |
| | | RU | 2015113767 | A | 10 December 2016 |
| | | RU | 2664465 | C2 | 17 August 2018 |
| | | TW | 201920098 | A | 01 June 2019 |
| | | TWI | 696611 | B | 21 June 2020 |
| | | ES | 2671644 | T3 | 07 June 2018 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| | | MX 2015003903 A | 17 July 2015 |
| | | MX 364484 B | 29 April 2019 |
| | | NZ 740948 A | 29 November 2019 |
| | | NZ 746439 A | 29 November 2019 |
| | | HK 1210477 A1 | 22 April 2016 |
| | | JP 2020180124 A | 05 November 2020 |
| | | JP 6952835 B2 | 27 October 2021 |
| | | EP 3342785 A1 | 04 July 2018 |
| | | EP 3342785 B1 | 25 December 2019 |
| | | IL 313147 A | 01 July 2024 |
| | | BR 122021014365 B1 | 05 July 2022 |
| | | KR 20220146669 A | 01 November 2022 |
| | | KR 102498405 B1 | 09 February 2023 |
| | | HUE 039000 T2 | 28 December 2018 |
| | | JP 2018188455 A | 29 November 2018 |
| | | JP 6715888 B2 | 01 July 2020 |
| | | KR 20230086800 A | 15 June 2023 |
| | | KR 102584005 B1 | 27 September 2023 |
| | | US 2019008981 A1 | 10 January 2019 |
| | | US 10973924 B2 | 13 April 2021 |
| | | PH 12015500667 A1 | 18 May 2015 |
| | | ZA 201900820 B | 31 August 2022 |
| | | LT 3342785 T | 10 February 2020 |
| | | MX 2019004502 A | 21 August 2019 |
| | | HUE 048748 T2 | 28 August 2020 |
| | | AU 2018200308 A1 | 01 February 2018 |
| | | AU 2018200308 B2 | 07 November 2019 |
| | | KR 20230024433 A | 20 February 2023 |
| | | KR 102540419 B1 | 05 June 2023 |
| | | RU 2018128384 A | 02 October 2018 |
| | | RU 2018128384 A3 | 20 December 2021 |
| | | AU 2022203560 A1 | 16 June 2022 |
| | | IL 294622 A | 01 September 2022 |
| | | IL 294622 B1 | 01 June 2023 |
| | | IL 294622 B2 | 01 October 2023 |
| | | HRP 20200353 T1 | 12 June 2020 |
| | | JP 2018008982 A | 18 January 2018 |
| | | JP 6371452 B2 | 08 August 2018 |
| CN 106163559 A | 23 November 2016 | MX 2016010533 A | 12 December 2016 |
| | | KR 20220068270 A | 25 May 2022 |
| | | KR 102445502 B1 | 21 September 2022 |
| | | KR 20200077620 A | 30 June 2020 |
| | | KR 102186027 B1 | 03 December 2020 |
| | | KR 20240008415 A | 18 January 2024 |
| | | TW 202428616 A | 16 July 2024 |
| | | CA 2939802 A1 | 15 October 2015 |
| | | CA 2939802 C | 01 November 2022 |
| | | JP 6148422 B1 | 14 June 2017 |
| | | JP 2017197515 A | 02 November 2017 |
| | | KR 20190004837 A | 14 January 2019 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| | | KR 102127623 B1 | 29 June 2020 |
| | | JP 2022180416 A | 06 December 2022 |
| | | JP 7383772 B2 | 20 November 2023 |
| | | RS 61711 B1 | 31 May 2021 |
| | | LT 3129063 T | 25 June 2021 |
| | | TW 201542230 A | 16 November 2015 |
| | | TWI 704928 B | 21 September 2020 |
| | | US 2019151328 A1 | 23 May 2019 |
| | | US 11298359 B2 | 12 April 2022 |
| | | HRP 20210593 T1 | 14 May 2021 |
| | | RU 2019143766 A | 20 February 2020 |
| | | KR 20200136052 A | 04 December 2020 |
| | | KR 102239413 B1 | 12 April 2021 |
| | | JP 2017503784 A | 02 February 2017 |
| | | JP 6105171 B2 | 29 March 2017 |
| | | KR 20210115056 A | 24 September 2021 |
| | | KR 102351755 B1 | 14 January 2022 |
| | | JP 2019135248 A | 15 August 2019 |
| | | JP 6707696 B2 | 10 June 2020 |
| | | IL 300540 A | 01 April 2023 |
| | | IL 300540 B1 | 01 April 2024 |
| | | IL 300540 B2 | 01 August 2024 |
| | | IL 248239 A0 | 30 November 2016 |
| | | IL 248239 B | 01 July 2022 |
| | | KR 20230021173 A | 13 February 2023 |
| | | KR 102624244 B1 | 11 January 2024 |
| | | TW 202108176 A | 01 March 2021 |
| | | TWI 745021 B | 01 November 2021 |
| | | SI 3129063 T1 | 31 August 2021 |
| | | US 2017021031 A1 | 26 January 2017 |
| | | US 10383878 B2 | 20 August 2019 |
| | | ES 2859648 T3 | 04 October 2021 |
| | | JP 2021169515 A | 28 October 2021 |
| | | JP 7138750 B2 | 16 September 2022 |
| | | AU 2020200227 A1 | 06 February 2020 |
| | | AU 2020200227 B2 | 07 October 2021 |
| | | KR 20210041126 A | 14 April 2021 |
| | | NZ 722668 A | 23 February 2024 |
| | | BR 112016017893 A2 | 17 October 2017 |
| | | BR 112016017893 B1 | 23 August 2022 |
| | | IL 311108 A | 01 April 2024 |
| | | SG 10201907807 XA | 27 September 2019 |
| | | CY 1124071 T1 | 27 May 2022 |
| | | EP 3129063 A1 | 15 February 2017 |
| | | EP 3129063 B1 | 27 January 2021 |
| | | IL 293177 A | 01 July 2022 |
| | | IL 293177 B1 | 01 March 2023 |
| | | IL 293177 B2 | 01 July 2023 |
| | | RU 2016143351 A | 15 May 2018 |
| | | RU 2016143351 A3 | 05 March 2019 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| | | RU 2711640 C2 | 17 January 2020 |
| | | PL 3129063 T3 | 12 July 2021 |
| | | DK 3129063 T3 | 06 April 2021 |
| | | EP 3789042 A1 | 10 March 2021 |
| | | EP 3789042 B1 | 06 November 2024 |
| | | SG 11201608309 PA | 29 November 2016 |
| | | KR 20160144396 A | 16 December 2016 |
| | | KR 101937549 B1 | 10 January 2019 |
| | | JP 2020143114 A | 10 September 2020 |
| | | JP 6918182 B2 | 11 August 2021 |
| | | JP 2024019172 A | 08 February 2024 |
| | | KR 20220012402 A | 03 February 2022 |
| | | KR 102399277 B1 | 18 May 2022 |
| | | HUE 054411 T2 | 28 September 2021 |
| | | MX 2021004884 A | 15 June 2021 |
| | | AU 2015245122 A1 | 18 August 2016 |
| | | AU 2015245122 B2 | 24 October 2019 |
| | | AU 2021286320 A1 | 20 January 2022 |
| | | AU 2021286320 B2 | 18 April 2024 |
| | | TW 202218685 A | 16 May 2022 |
| | | TWI 826828 B | 21 December 2023 |
| | | PH 12016501711 A1 | 19 December 2016 |
| | | WO 2015155998 A1 | 15 October 2015 |
| | | KR 20220132025 A | 29 September 2022 |
| | | KR 102495426 B1 | 06 February 2023 |
| | | AU 2024204502 A1 | 18 July 2024 |
| | | JP 2017222638 A | 21 December 2017 |
| | | JP 6513128 B2 | 15 May 2019 |
| | | PT 3129063 T | 01 April 2021 |
| | | MY 195180 A | 11 January 2023 |
| CN 110464847 A | 19 November 2019 | BR 112016013482 A2 | 03 October 2017 |
| | | BR 112016013482 B1 | 19 April 2022 |
| | | IL 278891 A | 31 January 2021 |
| | | IL 278891 B1 | 01 February 2024 |
| | | IL 278891 B2 | 01 June 2024 |
| | | KR 20210088745 A | 14 July 2021 |
| | | KR 102314913 B1 | 19 October 2021 |
| | | KR 20230162159 A | 28 November 2023 |
| | | KR 20220025176 A | 03 March 2022 |
| | | KR 102417312 B1 | 05 July 2022 |
| | | JP 2022180504 A | 06 December 2022 |
| | | JP 7467557 B2 | 15 April 2024 |
| | | PH 12016500904 A1 | 11 July 2016 |
| | | AU 2022203818 A1 | 23 June 2022 |
| | | AU 2020200596 A1 | 20 February 2020 |
| | | AU 2020200596 B2 | 03 March 2022 |
| | | TW 201834692 A | 01 October 2018 |
| | | TWI 661839 B | 11 June 2019 |
| | | HRP 20190431 T1 | 19 April 2019 |
| | | JP 2024081770 A | 18 June 2024 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| | | RU 2016123597 A | 02 March 2018 |
| | | RU 2016123597 A3 | 12 October 2018 |
| | | RU 2683780 C2 | 02 April 2019 |
| | | EP 3466976 A1 | 10 April 2019 |
| | | EP 3466976 B1 | 08 September 2021 |
| | | SG 10201800210 TA | 27 February 2018 |
| | | IL 310627 A | 01 April 2024 |
| | | LTPA 2021515 I1 | 10 August 2021 |
| | | LTC 3101032 I2 | 25 October 2022 |
| | | NL 301117 I1 | 19 July 2021 |
| | | NL 301117 I2 | 29 July 2021 |
| | | ES 2895254 T3 | 18 February 2022 |
| | | JP 2019112403 A | 11 July 2019 |
| | | JP 6665325 B2 | 13 March 2020 |
| | | CY 1121573 T1 | 29 May 2020 |
| | | LT 3101032 T | 25 March 2019 |
| | | EP 3101032 A1 | 07 December 2016 |
| | | EP 3101032 A4 | 06 September 2017 |
| | | EP 3101032 B1 | 16 January 2019 |
| | | US 2024026030 A1 | 25 January 2024 |
| | | TW 202237113 A | 01 October 2022 |
| | | TWI 796243 B | 11 March 2023 |
| | | TW 202322817 A | 16 June 2023 |
| | | TWI 848560 B | 11 July 2024 |
| | | TW 202237191 A | 01 October 2022 |
| | | TWI 796245 B | 11 March 2023 |
| | | US 2020385486 A1 | 10 December 2020 |
| | | US 11584800 B2 | 21 February 2023 |
| | | MX 2016006498 A | 04 August 2016 |
| | | KR 20210127803 A | 22 October 2021 |
| | | KR 102362920 B1 | 14 February 2022 |
| | | HUE 057464 T2 | 28 May 2022 |
| | | EP 4212552 A1 | 19 July 2023 |
| | | RS 58415 B1 | 30 April 2019 |
| | | JP 2021169493 A | 28 October 2021 |
| | | JP 7146031 B2 | 03 October 2022 |
| | | JP 2020097617 A | 25 June 2020 |
| | | JP 6914380 B2 | 04 August 2021 |
| | | EP 3973995 A1 | 30 March 2022 |
| | | FR 20C1030 I1 | 27 August 2021 |
| | | FR 20C1030 I2 | 08 April 2022 |
| | | HUE 044389 T2 | 28 October 2019 |
| | | SI 3101032 T1 | 28 February 2019 |
| | | RU 2019107788 A | 17 May 2019 |
| | | RS 62618 B1 | 31 December 2021 |
| | | WO 2015115091 A1 | 06 August 2015 |
| | | LT 3466976 T | 10 November 2021 |
| | | TW 202120088 A | 01 June 2021 |
| | | TWI 789700 B | 11 January 2023 |
| | | MX 2020010682 A | 06 November 2020 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | | | Publication date (day/month/year) |
|---|---|---|---|---|---|
| | | PT | 3466976 | T | 27 October 2021 |
| | | US | 2016333112 | A1 | 17 November 2016 |
| | | US | 10155821 | B2 | 18 December 2018 |
| | | ES | 2727351 | T3 | 15 October 2019 |
| | | TW | 201934145 | A | 01 September 2019 |
| | | TWI | 722422 | B | 21 March 2021 |
| | | KR | 20210040181 | A | 12 April 2021 |
| | | KR | 102275925 | B1 | 12 July 2021 |
| | | KR | 20220154251 | A | 21 November 2022 |
| | | KR | 102510128 | B1 | 14 March 2023 |
| | | SI | 3466976 | T1 | 31 December 2021 |
| | | JP | 6105183 | B1 | 29 March 2017 |
| | | JP | 2017105789 | A | 15 June 2017 |
| | | PT | 3101032 | T | 03 April 2019 |
| | | JP | 2017095457 | A | 01 June 2017 |
| | | JP | 6466895 | B2 | 06 February 2019 |
| | | NO | 2021027 | I1 | 06 July 2021 |
| | | KR | 20180122038 | A | 09 November 2018 |
| | | KR | 102190548 | B1 | 14 December 2020 |
| | | KR | 20230113822 | A | 01 August 2023 |
| | | KR | 102606930 | B1 | 29 November 2023 |
| | | TW | 201613649 | A | 16 April 2016 |
| | | TWI | 627967 | B | 01 July 2018 |
| | | HUS | 2100025 | I1 | 28 July 2021 |
| | | ZA | 201602802 | B | 27 September 2017 |
| | | HRP | 20211848 | T1 | 04 March 2022 |
| | | JP | 6046301 | B1 | 14 December 2016 |
| | | JP | 2017036285 | A | 16 February 2017 |
| | | NZ | 719202 | A | 25 February 2022 |
| | | CY | 2021018 | I2 | 15 October 2021 |
| | | CY | 1124774 | T1 | 25 November 2022 |
| | | KR | 20230042126 | A | 27 March 2023 |
| | | KR | 102557062 | B1 | 18 July 2023 |
| | | KR | 20220100994 | A | 18 July 2022 |
| | | KR | 102465042 | B1 | 09 November 2022 |
| | | IL | 266790 | A | 31 July 2019 |
| | | IL | 245252 | A0 | 30 June 2016 |
| | | IL | 245252 | B | 30 June 2019 |
| | | KR | 20200140932 | A | 16 December 2020 |
| | | KR | 102238848 | B1 | 09 April 2021 |
| | | DK | 3101032 | T3 | 29 April 2019 |
| | | TW | 202237114 | A | 01 October 2022 |
| | | TWI | 796244 | B | 11 March 2023 |
| | | JP | 5998289 | B2 | 28 September 2016 |
| | | JPWO | 2015115091 | A1 | 23 March 2017 |
| | | PL | 3101032 | T3 | 31 July 2019 |
| | | CA | 2928794 | A1 | 06 August 2015 |
| | | CA | 2928794 | C | 13 August 2019 |
| | | KR | 20160113099 | A | 28 September 2016 |
| | | KR | 101916553 | B1 | 07 November 2018 |

Form PCT/ISA/210 (patent family annex) (July 2022)

**INTERNATIONAL SEARCH REPORT**
**Information on patent family members**

International application No.
**PCT/CN2024/112677**

| Patent document cited in search report | Publication date (day/month/year) | Patent family member(s) | Publication date (day/month/year) |
|---|---|---|---|
| | | US 2019077880 A1 | 14 March 2019 |
| | | US 11795236 B2 | 24 October 2023 |
| | | TW 202237115 A | 01 October 2022 |
| | | TWI 796246 B | 11 March 2023 |
| | | PL 3466976 T3 | 20 December 2021 |
| | | AU 2015212265 B2 | 02 January 2020 |
| | | SG 11201603960 XA | 28 July 2016 |
| | | DK 3466976 T3 | 13 December 2021 |
| US 2017035906 A1 | 09 February 2017 | ES 2754348 T3 | 17 April 2020 |
| | | US 11185594 B2 | 30 November 2021 |
| | | WO 2015155976 A1 | 15 October 2015 |
| | | JP 2020079237 A | 28 May 2020 |
| | | JP 6861777 B2 | 21 April 2021 |
| | | JPWO 2015155976 A1 | 13 April 2017 |
| | | JP 6612738 B2 | 27 November 2019 |
| | | TW 201620554 A | 16 June 2016 |
| | | EP 3130608 A1 | 15 February 2017 |
| | | EP 3130608 A4 | 13 September 2017 |
| | | EP 3130608 B1 | 04 September 2019 |
| WO 2020240467 A1 | 03 December 2020 | BR 112021023901 A2 | 18 January 2022 |
| | | KR 20220015445 A | 08 February 2022 |
| | | TW 202108180 A | 01 March 2021 |
| | | SG 11202112429 PA | 30 December 2021 |
| | | EP 3976113 A1 | 06 April 2022 |
| | | CA 3142119 A1 | 03 December 2020 |
| | | JP 2022534725 A | 03 August 2022 |
| | | IL 288485 A | 01 January 2022 |
| | | US 2023270870 A1 | 31 August 2023 |
| | | AU 2020285681 A1 | 27 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311037231
- US 4816567 A
- CN 106163559 B


### Non-patent literature cited in the description


- **LI YUNFENG** ; **XIN JIE** ; **LI JING et al.** Research progress of linkage technology of antibody-drug conjugates[J].. *Chinese Journal of New Drugs and Clinical Remedies*, 2022, vol. 41 (12), 10
- **KHONGORZUL, PUREGMAA et al.** Antibody-Drug Conjugates: A Comprehensive Review. *Mol Cancer Res*, 2020, vol. 1
- **KOSTOVA VESELA** ; **DÉSOS PATRICE** ; **STARCK JÉRÔME-BENOÎT et al.** The Chemistry Behind ADCs.[J].. *Pharmaceuticals (Basel)*, 2021, vol. 14
- **DAN NIMOY** ; **SETUA SAINI** ; **KASHYAP VIVEK K et al.** Antibody-Drug Conjugates for Cancer Therapy: Chemistry to Clinical Implications.[J].. *Pharmaceuticals(Basel)*, 2018, vol. 11
- **CHOTHIA** ; **LESK**. *J. Mol. Biol.*, 1987, vol. 196, 901-917
- **CHOTHIA et al.** *Nature*, 1989, vol. 342, 878-883
- **EHRENMANN F** ; **KAAS Q** ; **LEFRANC M P.** IMGT/3Dstructure-DB and IMGT/DomainGapAlign: a database and a tool for immunoglobulins or antibodies, T cell receptors, MHC, IgSF and MhcSF [J].. *Nucleic acids research*, 2009, vol. 38 (suppl_1), D301-D307
- **EHRENMANN F** ; **KAAS Q** ; **LEFRANC M P.** IMGT/3Dstructure-DB and IMGT/DomainGapAlign: a database and a tool for immunoglobulins or antibodies, T cell receptors, MHC, IgSF and MhcSF [J].. *Nucleic acids research*, 2009, vol. 38 (suppl_1), D301-D307
- **KÖHLER G** ; **MILSTEIN C.** Continuous cultures of fused cells secreting antibody of predefined specificity [J].. *Nature*, 1975, vol. 256 (5517), 495
- **JONES et al.** *Nature*, 1986, vol. 321, 522-525
- **REICHMANN et al.** *Nature*, 1988, vol. 332, 323-329
- **PRESTA**. *Curr. Op. Struct. Biol.*, 1992, vol. 2, 593-596
- **CLARK**. *Immunol. Today*, 2000, vol. 21, 397-402
- **HOLLIGER P. et al.** *Proc. Natl. Acad. Sci. USA*, 1993, vol. 90, 6444-6448
- **POLJAK R.J. et al.** *Structure*, 1994, vol. 2, 1121-1123
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunology, Immunotherapy*, 2001, vol. 50 (1), 51-59
- Remington's Pharmaceutical Sciences. Mack Publishing Company, 1995
- **ACIERNO et al.** *J Mol Biol.*, 2007, vol. 374 (1), 130-46
- **HERMANSON, G. T**. Bioconjugate Techniques. Academic Press, 1996, 56-136, 456-493
- *Protein Science*, 1995, vol. 4, 2411-2423